(19) Europäisches Patentamt
European Patent Office
Office européen des brevets

(11) EP 2 488 509 B1

(12) # FASCICULE DE BREVET EUROPEEN

(45) Date de publication et mention de la délivrance du brevet:
**02.09.2015 Bulletin 2015/36**

(21) Numéro de dépôt: **09756765.5**

(22) Date de dépôt: **13.10.2009**

(51) Int Cl.:
***C07D 309/34*** (2006.01) ***C07D 335/02*** (2006.01)
***C07D 345/00*** (2006.01) ***C07D 409/14*** (2006.01)
***H01L 51/00*** (2006.01)

(86) Numéro de dépôt international:
**PCT/FR2009/001201**

(87) Numéro de publication internationale:
**WO 2011/045478 (21.04.2011 Gazette 2011/16)**

(54) **DERIVES DE TYPE DIPYRANNYLIDENE COMME COUCHE INTERFACIALE ANODIQUE DANS DES DISPOSITIFS ELECTRONIQUES**

DIPYRANNYLIDENDERIVATE ALS ANODENGRENZFLÄCHENSCHICHT BEI ELEKTRONISCHEN VORRICHTUNGEN

DIPYRANNYLIDENE DERIVATIVES AS AN ANODE INTERFACIAL LAYER IN ELECTRONIC DEVICES

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(43) Date de publication de la demande:
**22.08.2012 Bulletin 2012/34**

(73) Titulaires:
- **Centre National de la Recherche Scientifique (CNRS)
75016 Paris (FR)**
- **Université Pierre et Marie Curie (Paris 6)
75005 Paris (FR)**

(72) Inventeurs:
- **BERNY, Stéphane
F-91400 Orsay (FR)**
- **TORTECH, Ludovic
F-91190 Gif sur Yvette (FR)**
- **FICHOU, Denis
F-75014 Paris (FR)**

(74) Mandataire: **Mena, Sandra et al
Cabinet Orès
36, rue de St Pétersbourg
75008 Paris (FR)**

(56) Documents cités:

- **DATABASE CA [Online] CHEMICAL ABSTRACTS SERVICE, COLUMBUS, OHIO, US; 1986, MIURA, AKIRA ET AL: "Optical information recording material" XP002577132 extrait de STN Database accession no. 1986:216645 -& JP 60 264280 A (TOSHIBA CORP., JAPAN) 27 décembre 1985 (1985-12-27)**
- **BOLAG, ALTAN ET AL: "Field-Effect Transistors Based on Tetraphenyldipyranylidenes and the Sulfur Analogues" CHEMISTRY OF MATERIALS, vol. 21, no. 19, 28 août 2009 (2009-08-28) , pages 4350-4352, XP002577133**
- **AMATORE, CHRISTIAN ET AL: "Electrosynthesis of 2,2',6,6'-tetraaryl-4,4'-bipyranylidenes with eight flexible chains" TETRAHEDRON LETTERS, vol. 30, no. 11, 1989, pages 1383-1386, XP002577134**
- **REGNAULT DU MOTTIER, CHRISTINE; BRUTUS, MICHEL; COUSTUMER LE, GERARD; SAUVE, JEAN-PIERRE; EBEL, MAX; ET AL.: "Conducteurs Organiques: Synthese et Caracterisation de Complexes a Transfert de Charge de Bis(Chalcogenopyrannylidenes)-4:4' Avec le Tetracyanoquinodimethane" MOLECULAR CRYSTALS AND LIQUID CRYSTALS, vol. 154, 1988, pages 361-380, XP009131945**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 488 509 B1

Remarques:

Le dossier contient des informations techniques présentées postérieurement au dépôt de la demande et ne figurant pas dans le présent fascicule.

## Description

La présente invention est relative à des substrats revêtus de films comprenant des composés de formule (I), à leur procédé de fabrication et à leur utilisation comme couche interfaciale anodique dans des dispositifs électroniques. La présente invention concerne également des diodes électroluminescentes organiques (OLED), des diodes électroluminescentes polymériques (PLED), des transistors organiques à effet de champ (OFET) et des cellules solaires organiques (OSC) comprenant un substrat selon l'invention, des cellules solaires organiques spécifiques, ainsi que leur procédé de fabrication. Enfin, la présente invention concerne des composés de formule (I) en tant que tels.

Les systèmes électroniques actuels tels que les OSC nécessitent des technologies complexes qui rendent l'optimisation de leurs performances optique et électronique délicate.

Une des principales voies d'optimisation consiste à utiliser à l'anode du système photovoltaïque un polymère conducteur tel que le poly(3,4-éthylènedioxythiophène) - poly(styrènesulfonate) (PEDOT : PSS), ce dernier étant inséré entre une électrode d'oxyde d'indium dopé à l'étain (ITO) et une couche active photosensible (Crispin et al., Journal of Polymer Science: Part B: Polymer Physics, Vol. 41, 2561-2583 (2003)). Les substrats revêtus de PEDOT : PSS sont optiquement anisotropes et absorbent peu dans le visible ; la conductivité des substrats revêtus de PEDOT : PSS préparés au moyen de solutions commerciales est généralement comprise entre 1 et 50 S. $cm^{-1}$.

Les dispositifs à base de PEDOT : PSS font aujourd'hui encore l'objet de nombreux travaux (Groenendaal et al., Adv. Mater., 2000, 12, No. 7) relatifs à :

- l'amélioration de la couche active photosensible par l'utilisation de nouveaux matériaux absorbants, par la structuration de réseau de charges, ou encore par l'amélioration du transport des charges, et
- l'amélioration des interfaces grâce à une meilleure qualité des contacts ou une collecte des charges.

Ainsi, les couches interfaciales anodiques utilisées dans les dispositifs électroniques doivent présenter :

- de bonnes propriétés électroniques, et plus particulièrement une conductivité élevée, ainsi qu'un travail de sortie adapté permettant d'optimiser la barrière d'énergie à l'interface entre l'électrode d'ITO et la couche active photosensible, facilitant la collection des charges positives,
- de bonnes propriétés optiques se caractérisant par une absorption minimale, et
- une bonne stabilité et une facilité de mise en forme permettant la formation de films homogènes et continus.

On entend par travail de sortie l'énergie minimum, mesurée en électronvolts (eV), nécessaire pour arracher un électron depuis le niveau de Fermi d'un métal jusqu'à un point situé à l'infini en-dehors du métal. L'effet photoélectrique consiste en une libération d'un électron lorsqu'un photon doté d'une énergie supérieure au travail de sortie arrive sur le métal. La différence entre l'énergie du photon incident et le travail de sortie est fournie à l'électron sous forme d'énergie cinétique. Ainsi, le travail de sortie photoélectrique est calculé selon la formule :

$$\varphi = h.f_0$$

où $h$ est la constante de Planck et $f_0$ est la fréquence minimum du photon à partir de laquelle l'émission photoélectrique se produit.

Le travail de sortie des électrodes joue un rôle crucial dans le domaine de l'électronique plastique parce qu'il influe sur la distribution du champ électrique interne et la hauteur de la barrière d'énergie entre l'électrode et la couche active photosensible du dispositif. Cette barrière influence grandement l'injection des porteurs de charges, notamment dans le cas des OLED, ou au contraire, domine la collection des charges de la couche active vers l'électrode, comme par exemple dans le cas des OSC. Afin de faciliter le transport des trous, des matériaux présentant des travaux de sortie élevés sont préférés comme anode.

A l'heure actuelle, les dispositifs les plus couramment utilisés sont les dispositifs à base de PEDOT : PSS. Toutefois, lorsqu'il est utilisé comme couche interfaciale sur des électrodes d'ITO, le PEDOT : PSS doit être appliqué sous forme d'un film mince ayant une épaisseur inférieure à la centaine de nanomètres, ceci afin de garantir une transmission optique élevée ; le film de PEDOT : PSS présente dans ce cas une faible conductivité. Des défauts de surface et des trous peuvent également apparaître à la surface du film de PEDOT : PSS lorsque les couches de polymère appliquées sont trop fines. Par contre, lorsqu'il est appliqué en couche plus épaisse (entre 150 et 200 nm d'épaisseur), i. e. pour atteindre des conductivités plus élevées permettant un transport latéral du photocourant, on observe alors une perte au niveau du coefficient de transmission optique.

Les systèmes à base de PEDOT : PSS ont également d'autres inconvénients :

- l'interface entre les films de PEDOT : PSS et les électrodes à base d'ITO est instable, les atomes d'indium diffusant dans la couche polymère et altérant ses performances,
- le contact électrique entre les films de PEDOT : PSS et les électrodes à base d'ITO est faible, la couche de polymère n'accédant pas à de nombreux sites électroniquement actifs de la surface de l'électrode d'ITO, ce qui augmente les résistances de séries et diminue fortement la collection des trous à l'électrode,
- la conductivité et la rugosité des couches de PEDOT : PSS sont dépendantes des conditions d'application, et en particulier du taux d'humidité et des températures de recuit.

Des voies de substitution à l'utilisation de polymères conducteurs à l'interface anodique ont également été envisagées, comme par exemple :

- l'utilisation de monocouches (Campbell et al., Appl. Phys. Lett., 71 (24), 3528-3530; Kim et al., Appl. Phys. Lett., 92, 133307 (2008); Akkerman et al., Small 2008, 4, No. 1, 100-104; Armstrong et al., Thin Solid Films, 445, 2003, 342-352), ces dernières étant généralement liées de manière covalente par voie chimique ou électrochimique à la surface de l'électrode d'ITO. Toutefois, ces monocouches sont peu épaisses (quelques dizaines d'angströms) et conduisent à un recouvrement inégal des substrats qu'elles fonctionnalisent, laissant subsister des rugosités et conduisant ainsi à des interfaces de qualité médiocres,
- l'utilisation de complexes à transfert de charges (Hanson et al., J. Am. Chem. Soc., Vol. 127, No. 28, 2005, 127, 10058-10062; Kahn et al., Appl. Phys. Lett., Vol. 79, No. 24, 4040-4042), ces derniers présentant néanmoins l'inconvénient d'avoir des niveaux énergétiques difficilement ajustables.

Ainsi, le problème technique restant à résoudre par rapport à cet état de l'art consiste en la mise au point de substrats revêtus de films minces et homogènes, l'interface entre les couches et lesdits films devant être de grande qualité, posséder un degré de cristallinité élevé, et donc davantage de chemins de conduction pour la collecte de charges électriques positives (trous), en comparaison à ceux des films de polymères actuellement utilisés, les substrats revêtus de tels films pouvant ainsi être utilisés dans des dispositifs électroniques comme couche collectrice de trous, grâce à une mobilité des trous supérieure à celle des films de l'état de l'art, mais aussi à des niveaux énergétiques intermédiaires entre l'électrode anodique et la couche organique photosensible.

Les substrats de l'invention proposent de remédier aux inconvénients des polymères conducteurs actuels, grâce à un compromis de performances difficiles à atteindre répondant plus particulièrement aux besoins et exigences suivantes :

- une facilité de mise en oeuvre permettant la formation de substrats revêtus de films homogènes et continus sur l'électrode anodique, grâce à un taux de recouvrement élevé et à une bonne mouillabilité de la couche active photosensible, c'est-à-dire une bonne capacité à former un film homogène et continu sur la couche active photosensible, lesdits films ayant de préférence une épaisseur inférieure à 45 nm,
- une mobilité de trous élevée, c'est-à-dire une capacité intrinsèque à conduire les charges positives de la couche active photosensible à l'électrode d'ITO,
- une barrière énergétique abaissée, le niveau énergétique du film de l'invention devant être intermédiaire à ceux des matériaux qui l'entourent, favorisant la collection des charges positives à l'anode, et
- d'excellentes propriétés optiques, les films de l'invention possédant un coefficient de transmission élevé dans le visible,

toutes ces propriétés permettant d'augmenter les rendements de conversion des dispositifs électroniques dans lesquels les substrats de l'invention sont utilisés.

Cet objectif est atteint par les substrats revêtus de films comprenant au moins un composé de formule (I), tels que décrits ci-après, et qui constituent le premier objet de l'invention. La présente invention a également pour objet un procédé de fabrication de tels substrats, et l'utilisation de ces substrats comme couche interfaciale anodique dans des dispositifs électroniques. Font aussi partie de l'invention des diodes électroluminescentes organiques (OLED), des diodes électroluminescentes polymériques (PLED), des transistors organiques à effet de champ (OFET) et des cellules solaires organiques (OSC) comprenant un substrat selon l'invention, ainsi que des cellules solaires organiques spécifiques et leur procédé de fabrication. Enfin, un dernier objet de l'invention concerne des composés de formule (I) en tant que tels.

Ainsi, la présente invention a pour premier objet un substrat revêtu d'un film comprenant au moins un composé de formule (I) suivante :

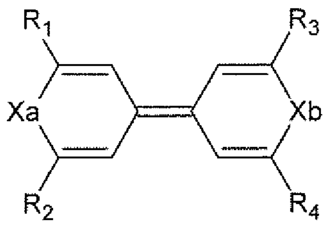

(I)

dans laquelle :

- Xa et Xb, identiques ou différents, sont choisis parmi les atomes N, P, O, S, Se ou Te,
- $R_1$, $R_2$, $R_3$ et $R_4$, identiques ou différents, représentent un groupement choisi parmi les cycles aryles ou hétéroaryles ayant 4 à 10 atomes de carbone, lesdits cycles aryles ou hétéroaryles pouvant éventuellement être substitués par un ou plusieurs atomes d'halogène, groupements hydroxyles -OH, alkyles ayant 1 à 30 atomes de carbone, alcoxy - $OC_nH_{2n+1}$ ou ester $-C(O)OC_nH_{2n+1}$, dans lesquels $0 \leq n \leq 30$, et de préférence $0 \leq n \leq 16$,

le composé de formule (I) étant présent dans ledit film sous forme de particules ayant un diamètre moyen inférieur ou égal à 300 nm, de préférence inférieur ou égal à 250 nm, et encore plus préférentiellement inférieur ou égal à 200 nm.

Le substrat de l'invention peut être un substrat à base d'oxyde, tel que l'oxyde d'indium dopé à l'étain (ITO), l'oxyde de fer, l'oxyde d'aluminium ou l'oxyde de silicium, revêtu d'un film tel que défini ci-dessus.

Ledit film consiste en une matrice dans laquelle des particules du composé de formule (I) sont réparties de façon sensiblement homogène, et constitue un matériau continu d'épaisseur mesurable. Ledit film présente, de préférence, une épaisseur inférieure à 45 nm, préférentiellement inférieure ou égale à 30 nm, et encore plus préférentiellement inférieure ou égale à 15 nm.

Des composés donneurs d'électrons tels que les composés de formule (I) de l'invention ont déjà été décrits dans l'art antérieur (Sandman et al., J. Chem. Soc., Chem. Commun., 1977, 687, 177-178; Otsubo et al., J. Chem. Soc. Perkin Trans., 1993, 1815-1824 ; Berenjian et al., Can. J. Chem., Vol. 59, 1981, 2612-2516), ces composés n'ayant toutefois, à ce jour, nullement été utilisés pour des applications électroniques telles que celles de l'invention.

Des semi-conducteurs organiques constitués de particules de 2,2',6,6'-tétraphényldipyran-4-ylidène ont également déjà été décrits dans l'art antérieur (Demande de brevet JP 60 264280 A ; Bolag et al., Chemistry of Materials, 2009, Vol-21, N°.19, 4350-4352) sans toutefois que ces particules ne présentent un diamètre moyen inférieur ou égal à 300 nm.

Au sens de la présente invention, on entend par :

- Alcoxy : un groupe O-alkyle dans lequel le groupe alkyle est un groupe aliphatique hydrocarboné saturé, linéaire ou ramifié ;
- Atome d'halogène : un atome de brome, de chlore, d'iode ou de fluore ; les désignations brome, chlore et fluore étant préférées ;
- Groupe aryle : désigne tout groupe fonctionnel ou substituant dérivé d'au moins un cycle aromatique ; un cycle aromatique correspond à tout groupe mono- ou polycyclique plan comportant un système $\pi$ délocalisé dans lequel chaque atome du cycle comporte une orbitale p, lesdites orbitales p se recouvrant les unes aux autres ;
- Groupe hétéroaryle : désigne tout groupe fonctionnel ou substituant dérivé d'au moins un cycle aromatique tel que défini ci-dessus et contenant au moins un hétéroatome choisi parmi N, P, O et S.

Selon un mode de réalisation préféré de l'invention, les atomes Xa et Xb du composé de formule (I) sont identiques et choisis parmi les atomes O, S ou Se, et de préférence Xa = Xb = S.

De manière avantageuse, les cycles aryles ou hétéroaryles $R_1$, $R_2$, $R_3$ et $R_4$ ayant 4 à 10 atomes de carbones sont choisis parmi les cycles phényle, naphtyle, anthracyle, benzoxazolyle, thiophényle ou alcoxy-thiophényle, furyle, pyrrolyle, pyridyle, pyrazyle, pyrazolyle, pyridazyle, pyrimidyle, triazyle, imidazolyle, oxazolyle, indyle, indazolyle, quinolyle et quinoxalyle.

De préférence, lesdits cycles sont identiques et choisis parmi les cycles phényle, naphtyle ou alcoxy-thiophényle suivants :

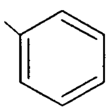 ; 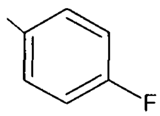 ; 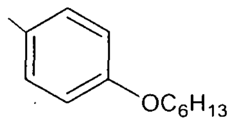 ; 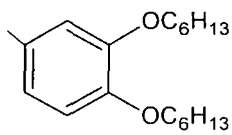 ;

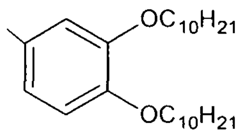 ; 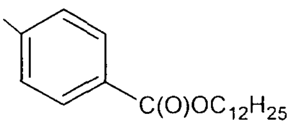 ; 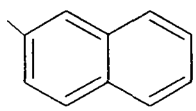 ; 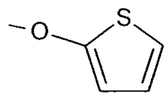

De manière encore plus avantageuse, le substrat de l'invention est revêtu d'un film comprenant au moins un composé de formule (I) choisi parmi les composés suivants :

- Composé (1) :

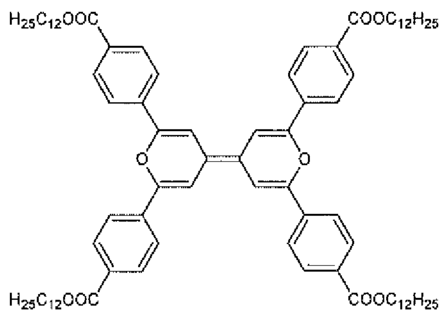

- Composé (2) :

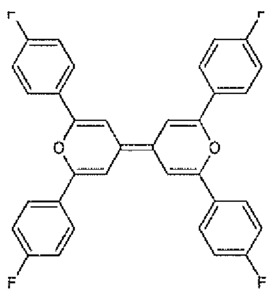

- Composé (3) :

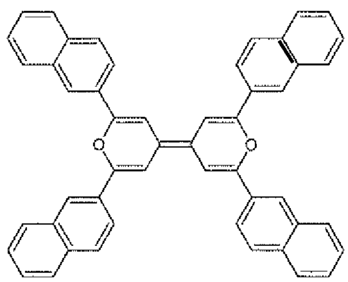

- Composé (4) :

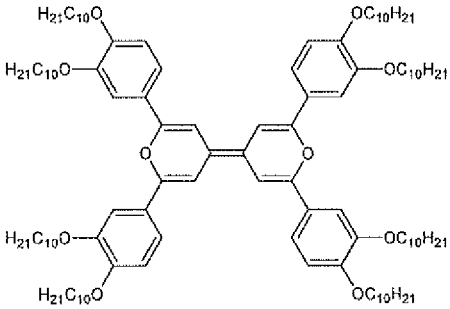

- Composé (5) :

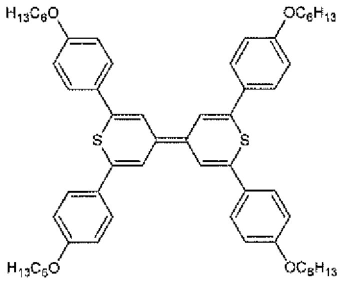

- Composé (6) :

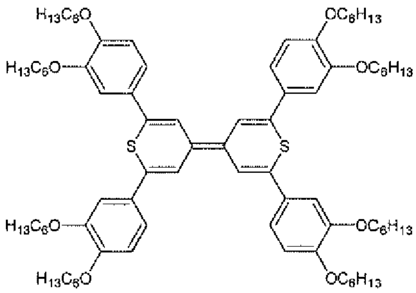

- Composé (7) :

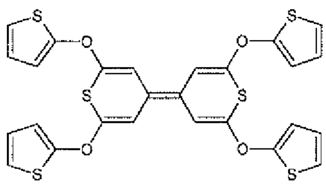

- Composé (8) :

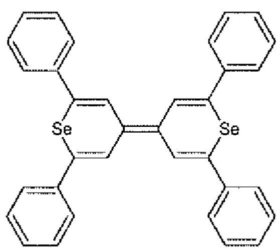

En plus du composé de formule (I), le film de l'invention peut également comprendre d'autres constituants, et plus particulièrement des molécules acceptrices d'électrons conduisant à la formation de complexes de transfert de

charges, telles que le 7,7,8,8-tétracyana-*p*-quinadiméthane (TCNQ) ou le tétrafluoro-7,7,8,8-tétracyana-*p*-quinodiméthane ($F_4$TCNQ). Ces molécules acceptrices d'électrons sont introduites en faible quantité par rapport au composé de formule (I). De préférence, le ratio en poids molécule accepteuses d'électrons/composés de formule (I) est compris entre 1/99 et 5/95. Et elles agissent comme des agents dopants en augmentant les propriétés intrinsèques de conduction des trous du composé de formule (I).

La présente invention a également pour objet un procédé de fabrication d'un substrat tel que défini selon l'invention comprenant au moins une étape de dépôt par voie sèche d'un film comprenant un composé de formule (I) tel que défini ci-dessus, ledit dépôt étant réalisé à une vitesse inférieure à 1 Å/s, de préférence inférieure ou égale à 0,4 Å/s, et encore plus préférentiellement inférieure ou égale à d,1 Å/s.

Le dépôt par voie sèche est réalisé par chauffage sous vide ; il permet de sublimer le composé de formule (I). Il peut être réalisé par un dépôt chimique en phase vapeur CVD, cette technique consistant à ajouter un précurseur métallique à une source de carbone liquide (telle que le toluène, le benzène ou le cyclohexane), la solution étant alors transformée en fines gouttelettes, transportées par un gaz inerte jusqu'à un four. En pratique, le composé de formule (I) est placé, sous forme de poudre, dans un creuset, puis recouvert d'alumine, et ensuite chauffé sous vide jusqu'à ce que le composé de formule (I) passe de l'état solide à l'état liquide (sublimation). Le flux de vapeur du composé de formule (I) est alors orienté vers le substrat de manière à le recouvrir d'un film constitué du composé de formule (I).

Le chauffage sous vide, nécessaire pour sublimer le composé de formule (I), est de préférence réalisé à une température comprise entre 80 et 180°C, et encore plus préférentiellement à une température approximative de 150°C, sous une pression comprise entre $10^{-4}$ et $10^{-8}$ mbar, et encore plus préférentiellement sous une pression proche de $10^{-6}$ mbar, pendant une durée comprise entre 2 et 4 heures, et encore plus préférentiellement pendant une durée approximative de 3 heures.

Le substrat revêtu obtenu selon ce procédé est constitué d'un film homogène de grande qualité, possédant un degré de cristallinité élevé. En effet, le film de l'invention est constitué d'une répétition quasi-identique de motifs (ou grains de matière), répartis de manière continue sur l'intégralité du substrat, l'interface électrique entre le substrat et le film étant constituée de nombreux chemins de conduction des charges positives.

Un autre objet de l'invention concerne l'utilisation d'un substrat selon l'invention comme couche interfaciale anodique, et plus particulièrement comme couche collectrice de trous, dans des dispositifs électroniques tels que les diodes électroluminescentes organiques (OLED), les diodes électroluminescentes polymériques (PLED), les transistors organiques à effet de champ (OFET) et les cellules solaires organiques (OSC), l'utilisation comme anode dans les OSC étant particulièrement préférée.

Un objet supplémentaire de l'invention concerne un article fini choisi parmi les OLED, les PLED, les OFET et les OSC comprenant au moins un substrat selon l'invention. Plus particulièrement, la présente invention a également pour objet une cellule solaire organique (OSC), telle que définie ci-dessus, dans laquelle le substrat est à base d'oxyde, et préférentiellement à base d'ITO.

De manière préférée, la cellule solaire organique (OSC) de l'invention comprend au moins un support de base (a) revêtu d'un substrat (b) tel que défini selon l'invention, ledit substrat (b) étant lui-même revêtu d'une couche active photosensible (c).

Le support de base (a) peut être rigide ou souple. Il est de préférence choisi parmi le verre, les métaux ou leurs oxydes, et les polymères. De préférence, le support de base (a) est un oxyde métallique choisi parmi l'oxyde d'aluminium, l'oxyde d'indium, l'oxyde d'étain, l'oxyde d'iridium, l'oxyde de silicium, l'oxyde de fer et l'oxyde de cuivre, ou un polymère choisi parmi le polyéthylène téréphtalate (PET), le polyéthylène amorphe (PE amorphe) et le polystyrène amorphe (PS amorphe).

La couche active photosensible (c) est une couche constituée d'au moins deux matériaux fortement absorbants dans le visible, l'un étant donneur d'électrons et l'autre accepteur d'électrons, cette couche subissant un transfert de charges ultra-rapide et créant des charges positives (trous) et négatives (électrons) sous l'effet d'une irradiation lumineuse. Cette couche active photosensible (c) peut être l'association de dérivés de phtalocyanines, de pentacène, de thiophène, de triphénylamine, de polymères de type p, avec des molécules telles que les dérivés des fullerènes ou les pérylènes, le mélange $P_3HT$ : PCBM étant l'association préférée.

Selon un mode de réalisation avantageux, le support de base (a) est en verre, le substrat (b) de l'invention est à base d'ITO, et la couche active photosensible (c) est à base de $P_3HT$ : PCBM.

La couche active photosensible (c) peut également être revêtue d'une couche (d), également appelée couche active de dissociation des excitons (ou EBL pour Exciton Blocking Layer), pouvant être à base de fluorure de lithium (LiF) dans les dispositifs OSC ou à base d'alumine dans les dispositifs OLED ou PLED.

La couche (d) peut elle aussi être revêtue d'une couche électrolytique (e) à base d'aluminium, d'or, de calcium, de cuivre, de samarium, de platine, de palladium, de chrome, de cobalt ou d'iridium, la couche (e) étant de préférence à base d'aluminium.

La présente invention a également pour objet un procédé de fabrication d'une cellule solaire organique (OSC) selon l'invention comprenant au moins les étapes suivantes :

(i) le dépôt d'un substrat (b) tel que défini selon l'invention sur un support de base (a), puis
(ii) le dépôt d'une couche active photosensible (c), ledit dépôt étant de préférence réalisé à la tournette,
(iii) éventuellement, le recuit de la couche active photosensible (c) dans un four tubulaire, à une température comprise entre 30 et 150°C, de préférence entre 80 et 150°C, et encore plus préférentiellement proche de 120°C, pendant une durée comprise entre 1 minute et 24 heures, de préférence entre 5 à 20 minutes, et encore plus préférentiellement pendant approximativement 10 minutes, sous une atmosphère inerte d'argon,
(iv) éventuellement, le dépôt d'une couche (d) à base de fluorure de lithium (LiF), ledit dépôt pouvant être réalisé par voie sèche, à une vitesse d'évaporation inférieure à 1 Å/s, de préférence inférieure ou égale à 0,4 Å/s, et encore plus préférentiellement inférieure à 0,1 Å/s,
(v) éventuellement, le dépôt d'une couche électrolytique (e), ledit dépôt pouvant être réalisé par voie sèche, à une vitesse d'évaporation inférieure ou égale à 3 Å/s, et de préférence comprise entre 1 et 2 Å/s.

De manière avantageuse, la couche (d) présente une épaisseur inférieure à 10 Å, de préférence inférieure à 8 Å, et encore plus préférentiellement comprise entre 5 et 7 Å.

Enfin, la présente invention protège également des composés de formule (I) en tant que tels :

- Composé (1) :

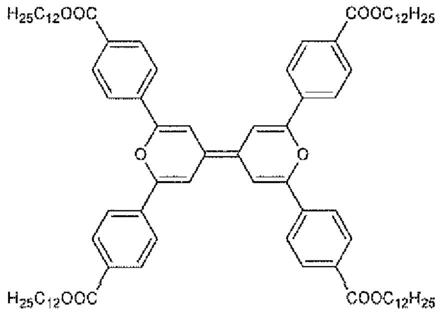

- Composé (3) :

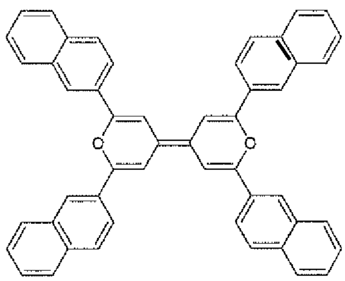

- Composé (5) :

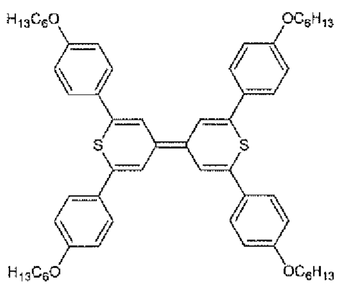

- Composé (6) :

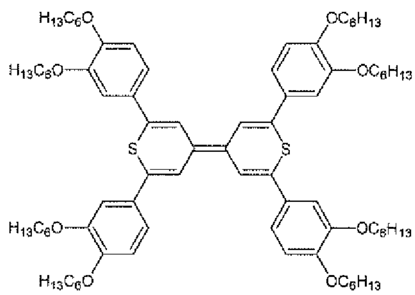

- Composé (7) :

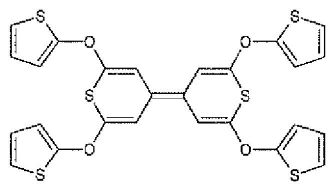

Les composés de formule (I) peuvent être synthétisés selon des méthodes connues de l'homme du métier, telles que celles décrites par Sandman et al., J. Chem. Soc., Chem. Commun., 1977, 687, 177-178, et Otsubo et al., J. Chem. Soc. Perkin Trans., 1993, 1815-1824.

Outre les dispositions qui précèdent, l'invention comprend encore d'autres dispositions qui ressortiront de la description qui va suivre, qui se réfère à des exemples de mise en oeuvre et d'évaluation de substrats revêtus de films comprenant au moins un composé de formule (I) selon l'invention, ainsi qu'aux dessins annexés dans lesquels :

- La **Figure 1** illustre des images capturées au microscope optique (grossissement x 5) de couches préparées par voie humide dans différents solvants : a) dans du dichlorométhane, b) dans du toluène, c) dans du benzène, d) dans du xylène, e) dans du chloroforme, et f) dans du tétrahydrofurane,
- la **Figure 2** illustre des images capturées au Microscope à Force Atomique (AFM) en mode *« Détection de courant »* de couches préparées selon différents régimes d'évaporation : a) en régime d'évaporation instable (5 μm x 5 μm), b) en régime d'évaporation stable (5 μm x 5 (μm), c) en régime d'évaporation stable (2 μm x 2 μm), et d) image de phase, en régime d'évaporation stable (2 μm x 2 μm),
- la **Figure 3** représente le spectre d'absorption de substrats revêtus de films à base de 2,2',6,6'-tétraphényl-4,4'-dithiopyrannylidène, ayant des épaisseurs différentes,
- la **Figure 4** représente le spectre d'absorption d'un film de 45 nm d'épaisseur à base de 2,2',6,6'-tétraphényl-4,4'-dithiopyrannylidène, et d'un film de 20 nm d'épaisseur à base de PEDOT : PSS,
- la **Figure 5** représente le diagramme énergétique des différentes couches présentes dans un dispositif photovoltaïque,
- la **Figure 6** illustre des images réalisées par AFM en mode *« Détection de courant »* d'un film à base de 2,2',6,6'-tétraphényl-4,4'-dithiopyrannylidène et d'un film à base de PEDOT : PSS, pour une différence de potentiel de -600 mV,
- la **Figure 7** représente l'évolution du courant en fonction du champ électrique appliquée dans une couche de $P_3HT$,
- la **Figure 8** représente l'évolution du courant en fonction de la tension appliquée d'un film selon l'invention et d'un film de référence à base de $P_3HT$, ces mesures ayant été déterminées au Microscope à Force Atomique en détection de courant (CS-AFM),
- la **Figure 9** représente la mobilité des charges positives d'un film selon l'invention et d'un film de référence à base de $P_3HT$,
- la **Figure 10** schématise un dispositif OSC de référence comprenant une interface d'ITO recouverte d'un polymère conducteur à base de PEDOT : PSS et d'une couche active photosensible à base de $P_3HT$-PCBM,
- la **Figure 11** précise sur un graphe J = f(V) la manière dont ont été déterminés les caractéristiques principales des dispositifs photovoltaïques,
- la **Figure 12** représente les courbes I-V d'un dispositif non recuit selon l'invention et de deux dispositifs de référence non recuits, sous éclairement,
- la **Figure 13** représente les courbes de réponse du photocourant de différentes diodes, sous éclairement,
- la **Figure 14** représente les courbes I-V de dispositifs comprenant des substrats revêtus de films de différentes épaisseurs, sous éclairement,

- la **Figure 15** représente les courbes I-V d'un dispositif recuit selon l'invention et de deux dispositifs de référence recuits, sous éclairement,
- la **Figure 16** représente des images réalisées par AFM en mode « *Détection de courant* » d'un film à base de 2,2',6,6'-tétraphényl-4,4'-dithiopyrannylidène de 14 nm d'épaisseur [image a) (5 μm x 5 μm) et image d) (2 μm x 2 μm)], d'un film à base de PEDOT : PSS de 5 nm d'épaisseur [image b) (5 μm x 5 μm) et image e) (2 μm x 2 μm)], et d'un film « hybride » comprenant une couche à base de 2,2',6,6'-tétraphényl-4,4'-dithiopyrannylidène (14 nm d'épaisseur), recouverte d'une couche de PEDOT : PSS (5 nm d'épaisseur) [image c) (5 μm x 5 μm) et image f) (2 μm x 2 μm)].

**Exemples :**

Un substrat revêtu d'un film comprenant, en tant que composé de formule (I), du 2,2',6,6'-tétraphényl-4,4'-dithiopyrannylidène, a été préparé selon le mode opératoire décrit ci-après.

La formule du 2,2',6,6'-tétraphényl-4,4'-dithiopyrannylidène est la suivante :

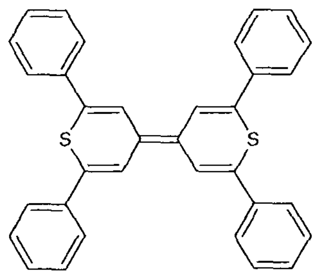

A- **Synthèse du 2,2',6,6'-tétraphényl-4,4'-dithiopyrannylidène (composé (I) selon l'invention)**

Etape 1 : synthèse du 1,5-diphényle-1,5-pentadione

Dans un ballon bicol de 100 mL, une solution de chlorure de glutaryle (10 mmol, 1,3 mL) solubilisée dans du dichlorométhane anhydre (20 mL) est introduite sous atmosphère inerte. Le chlorure d'aluminium (30 mmol, 4,00 g) est additionné au mélange, qui se colore immédiatement en jaune-orangée. Une solution de benzène (20 mmol, 1,8 mL) solubilisée dans du dichlorométhane anhydre (10 mL) est additionnée goutte à goutte à température ambiante. La solution devient rouge intense. Le mélange est ensuite porté à reflux pendant 24 heures, et se colore en marron-noir. Le mélange est ensuite ramené à température ambiante, puis versé dans un cristallisoir contenant 20 mL d'eau acidifiée à 10%, sous agitation. Un solide noir se forme, puis est filtré sur Büchner. La phase organique de couleur jaune est ensuite extraite à l'acétate d'éthyle, séchée sur $MgSO_4$, filtrée, concentrée à l'évaporateur rotatif et recristallisé dans 20 mL de méthanol. Le produit obtenu est un solide blanc de masse 680 mg. Le rendement de la réaction est de 27%.
CCM (éluant : éther de pétrole/ acétate d'éthyle 1:1) : 0,75
$^1$H RMN ($CDCl_3$, 400 MHz) : $\delta$ = 7.98 (d, 4H, $^3$J = 7,1 Hz), 7,57 (t, 2H, $^3$J = 7,3 Hz), 7,47 (t, 4H, $^3$J = 7,3 Hz), 3,13 (t, 4H, $^3$J = 6,9 Hz), 2,21 (quint, 2H, $^3$J = 6,9 Hz)

Etape 2 : synthèse du perchlorate de diphényle-2,6-thiopyrylium

Dans un ballon bicol de 100 mL, le 1,5-diphényle-1,5-pentadione préparé lors de l'étape 1 (10 mmol, 2,52 g) est mélangé à du pentasulfure de phosphore (15 mmol, 3,34 g), de l'acide acétique (60 mL) et du perchlorate de lithium (60 mmol, 6,40 g), puis porté à reflux pendant 3 heures. La solution devient alors orange et un précipité blanc se forme. Le précipité est éliminé par filtration sur fritté, puis lavé à l'acide acétique chaud. La solution est concentrée à l'évaporateur rotatif et recristallisée par addition d'éther (500 mL). Le précipité obtenu est filtré sur fritté, puis séché à l'évaporateur rotatif. Le produit obtenu est un solide jaune de masse 850 mg. Le rendement de la réaction est de 24%.
CCM (éluant : éther de pétrole/ acétate d'éthyle 8:2) : 0,0
$^1$H RMN ($D_2O$, 400 MHz) : $\delta$ = 8,80 (m, 3H), 7,92 (d, 4H, $^3$J = 7,3 Hz), 7,68 (t, 2H, $^3$J = 7,3 Hz), 7,60 (t, 4H, $^3$J = 8,0 Hz)

Etape 3 : synthèse du 2,2',6,6'-tétraphényl-4,4'-dithiopyrannylidène

Dans un ballon bicol de 250 mL, la solution de perchlorate de diphényle-2,6-thiopyrylium (10 mmol, 3,50 g) est solubilisée dans 200 mL d'acétonitrile distillée, puis le mélange est porté à reflux pendant 2 heures sous atmosphère inerte. Du zinc en poudre (30 mmol, 1,96 g) est additionné par petites fractions, et le mélange est à nouveau porté à

reflux pendant 24 heures. La solution est filtrée, rincée avec du toluène et évaporée à l'évaporateur rotatif. On obtient une huile noire qui est recristallisée dans un mélange hexane/éthanol 1:1. Un solide noir de masse 650 mg est obtenu. Le rendement de la réaction est de 13%.
CCM (éluant : éther de pétrole/ acétate d'éthyle 8:2) : 0,64
IR ($cm^{-1}$) : 3018, 1735, 1570, 1488, 1441, 1229, 1071, 750, 682

**B- Fabrication et mise en oeuvre des substrats de l'invention**

Le 2,2',6,6'-tétraphényl-4,4'-dithiopyrannylidène a été appliqué sur une électrode d'ITO, elle-même appliquée sur un support de base en verre, selon deux techniques différentes :

- un dépôt par voie humide, et
- un dépôt par voie sèche.

Des images de ces substrats, capturées au microscope optique et par AFM, sont représentées aux Figures 1 et 2.
Le dépôt par voie humide a été réalisé à la tournette (« *spin coating* ») dans différents solvants, en déposant une solution concentrée en composé de formule (I) sur un substrat en rotation à 2000 tours/min pendant 50 secondes. Du fait de la faible solubilité du 2,2',6,6'-tétraphényl-4,4'-dithiopyrannylidène dans les solvants usuels, les films formés et évalués étaient inhomogènes (présence d'aiguilles de 50 nm à 300 $\mu$m de longueur et de 1 à 15 $\mu$m de diamètre - cf. **Figure 1**), et ce quelques soient les conditions utilisées (concentration, nature du solvant, méthode de préparation).
Le dépôt par voie sèche a été réalisé par MO-CVD (Metal Organic - Chemical Vapor Deposition). Les films obtenus ont été observés par AFM et sont représentés à la **Figure 2**. La morphologie des films obtenus est fortement influencée par le régime d'évaporation. Les dépôts les plus homogènes sont obtenus pour des vitesses d'évaporation stables (0,1 Å/s). Les films présentent des distributions « mono-disperses » de particules de 2,2',6,6'-tétraphényl-4,4'-dithiopyrannylidène ayant un diamètre moyen d'environ 200 nm. Le régime d'évaporation est considéré comme instable lorsque la vitesse d'évaporation durant l'application est, à un moment donné, supérieure à 1 Å/s.
Le Tableau I ci-dessous illustre les caractéristiques de plusieurs substrats revêtus de films comprenant du 2,2',6,6'-tétraphényl-4,4'-dithiopyrannylidène, montrant l'influence de l'épaisseur et du régime d'évaporation (dépôt par voie sèche) sur la rugosité RMS (« Root Mean Square ») des films.

**Tableau I :**

| Régimes d'évaporation | Epaisseurs des films | Rugosités RMS |
|---|---|---|
| **Stable (vitesse d'évaporation = 0,1 Å/s)** | 14 | 5,5 |
| | 18 | 5,1 |
| | 25 | 9,05 |
| | 45,1 | 12,5 |
| **Instable (vitesse d'évaporation = 1,5 Å/s)** | 37 | 18,7 |

La rugosité RMS correspond à la mesure des hauteurs de l'ensemble des grains présents dans le film (accessibles par AFM), par rapport à la moyenne de ces valeurs : c'est un écart-type à la moyenne des hauteurs. Plus cette valeur tend vers 0, plus les hauteurs des grains sont proches les unes des autres, et plus le film est considéré comme homogène.
En conclusion, un dépôt par voie sèche en régime d'évaporation stable permet d'obtenir des couches thermiquement polycristallines et mono-disperses.

**C- Evaluation des propriétés optiques des substrats de l'invention**

Des spectres d'absorption obtenus pour différentes épaisseurs de films sont représentés à la **Figure 3**.
Les spectres d'absorption obtenus présentent deux maximas situés respectivement à 530 nm et 595 nm. Le premier maxima correspond à une transition $\pi \rightarrow \pi^*$, alors que le second maxima semble mettre en évidence un transfert de charges interne dans la couche organique de 2,2',6,6'-tétraphényl-4,4'-dithiopyrannylidène, mettant ainsi en évidence le haut degré de cristallinité du film formé.
On observe que l'absorption des couches augmente avec leur épaisseur (on confirme ainsi qualitativement les mesures d'épaisseurs déterminées par AFM). Un écart à cette loi s'observe pour le film ayant une épaisseur de 37 nm

issu d'une évaporation selon un régime instable.

Le spectre d'absorption d'un film selon l'invention a également été comparé au spectre d'absorption d'un film de PEDOT : PSS représentatif de l'état de l'art (cf. Figure 4). L'absorption du film de l'invention est élevé et beaucoup plus intense que celle du film de PEDOT : PSS, notamment pour des longueurs d'onde situées dans le visible.

**D- Evaluation des propriétés électroniques des substrats de l'invention**

Les niveaux énergétiques de la molécule de 2,2',6,6'-tétraphényl-4,4'-dithiopyrannylidène ont été déterminés par voltamérie cyclique. Ces mesures ont été réalisées à une vitesse de balayage de 0,1 V/s. L'électrode de travail utilisée est en platine. Les mesures de potentiel ont été effectuées par rapport à une électrode de référence au calomel saturée.

Le 2,2',6,6'-tétraphényl-4,4'-dithiopyrannylidène présente deux vagues réversibles d'oxydation situées respectivement à 330 mV et à 550 mV. Le fait que les vagues d'oxydation soient réversibles suggère que la molécule oxydée est stable durant toute la durée de la mesure.

Les valeurs des vagues d'oxydation obtenues sont assez faibles et l'écart entre les deux pics d'oxydation est de 220 mV, ce qui nous permet de déterminer le niveau HOMO de la molécule (niveau énergétique de la plus haute orbitale moléculaire occupée) selon deux méthodes différentes *(*D'Andrade et al., Organic Electronics 6, 2005, 11-20) :

Formule 1 : $E_{HOMO}$ = -(1,4 +/- 0,1)*q*Vcv - (4,6 +/- 0,08)
$E_{HOMO}$ = (-4,806 / -5,01) eV
dans laquelle :

**-**q correspond à la charge électronique et est égale à 1, et
**-**Vcv correspond à la différence entre deux potentiels d'oxydation et est égale à 0,22 eV

Formule 2 : $E_{HOMO}$ = -4,72 - (valeur du premier potentiel d'oxydation)
$E_{HOMO}$ = -5,05 eV

Le tableau II ci-dessous rassemble l'ensemble des valeurs énergétiques obtenues pour le 2,2',6,6'-tétraphényl-4,4'-dithiopyrannylidène (selon différentes techniques, la technique UPS signifiant « Ultraviolet Photoemission Spectroscopy »).

**Tableau II :**

| Couche interfaciale anodique | Technique de mesure | HOMO (eV) | LUMO (eV) | Ecart (eV) |
|---|---|---|---|---|
| 2,2',6,6'-tétraphényl-4,4'-dithiopyrannylidène isolé (en solution dans du tétrahydrofurane) | Modélisation moléculaire | -4,38 | -1,90 | 2,48 |
| | Absorption UV-Visible sur solution | - | - | 2,62 |
| | Voltamétrie cyclique (Formule 1) | -4,8 / -5,0 | - | - |
| | Voltamétrie cyclique (Formule 2) | -5,05 | - | - |
| Film à base de 2,2',6,6'-tétraphényl-4,4'-dithiopyrannylidène | Absorption UV-Visible sur film | - | - | 2,08 |
| | UPS | -4,8 / -4,9 | - | - |
| ITO | UPS | -4,45 / -4,55 | - | - |
| PEDOT : PSS | UPS | -5,2 | - | - |

Les différentes valeurs obtenues ont permis de dresser le diagramme énergétique de la **Figure 5**.

On constate alors que le 2,2',6,6'-tétraphényl-4,4'-dithiopyrannylidène possède des niveaux énergétiques intermédiaires entre le niveau de Fermi de l'ITO et celui du poly(3-hexylthiophène) ($P_3HT$) et du [6,6]-phényl-$C_{61}$-butyrate de méthyle (PCBM), le $P_3HT$/ PCBM constituant la couche active photosensible. La barrière énergétique permettant le passage de charges positives est abaissée à l'anode, ce qui en fait un excellent candidat pour la collection de trous dans des dispositifs photovoltaïques.

Les conditions énergétiques étant favorables à l'utilisation de dérivés de type dipyrannylidène à l'interface anodique, la capacité du 2,2',6,6'-tétraphényl-4,4'-dithiopyrannylidène à conduire les charges (mobilités de trous) a également été évaluée afin d'attester de l'éventuelle efficacité de cette interface.

Etude de l'injection de charges dans les films de 2,2',6,6'-tétraphényl-4,4'-dithiopyrannylidène par AFM en détection de courant :

La compréhension des effets de la structuration des surfaces employées dans les dispositifs photovoltaïques a été étudiée par Microscopie à Force Atomique en détection de courant (CS-AFM), cette dernière permettant concomitamment de mesurer les propriétés de transport de charges vertical par des mesures locales de courant-tension (I-V) et de capturer les images de topographie des couches étudiées.

*a) Principe des mesures en sonde locale CS-AFM*

Toutes les mesures ont été réalisées au moyen d'un microscope PicoLE (Molecular Imaging), équipé d'un nez permettant la détection de courant (gain du préamplificateur = 1 nA/V et gamme de courant +/- 10 nA). Pour ces mesures, le microscope travaille en mode « *Contact ».*

Les pointes utilisées présentent un revêtement conducteur de $PtIr_5$ avec une constante de raideur de 0,2 N/m et un rayon de 25 nm, et sont connectées à une masse virtuelle via le préamplificateur.

Pour les mesures, la pointe conductrice utilisée est en contact avec la surface du substrat (la pointe agit en fait comme une nano-électrode) et mesure la réponse du courant en fonction de la tension appliquée en différents points du substrat (courbes I-V). La tension est appliquée au substrat conducteur d'ITO et le courant est mesuré via le préamplificateur. Pour chacun des substrats, plus de 200 courbes I-V sont collectées en différents endroits afin d'établir une moyenne des résultats.

La morphologie des couches de 2,2',6,6'-tétraphényl-4,4'-dithiopyrannylidène a ainsi pu être corrélée au régime de transport de charges, et les mobilités de charges du film ont pu être déterminées par comparaison avec une référence connue, i.e. le $P_3HT$.

Deux types de couches ont été étudiés :

- une couche de $P_3HT$ de 47 nm d'épaisseur, qui sert de référence pour le calcul des mobilités, cette dernière ayant été obtenue à partir d'une solution de $P_3HT$ à 15 mg/mL dans du chlorobenzène. Pour cela, 15 mg de poudre de $P_3HT$ sont solubilisés dans 1 mL de chlorobenzène, le solvant est ensuite filtré sur un filtre Téflon® ayant des pores de 200 $\mu$m de diamètre, et la solution de $P_3HT$ est placée dans un bain à sonification pendant 2 heures, à température ambiante. 10 $\mu$L de cette solution sont ensuite déposés à la tournette (« *spin coating* ») sur un substrat d'ITO de dimension 1 cm x 2,5 cm (à une vitesse de 2000 tours/min pendant 50 secondes). Aucun recuit n'a été réalisé sur ce substrat et aucune couche de PEDOT : PSS n'a été déposée entre le substrat d'ITO et la couche de $P_3HT$. L'épaisseur de 47 nm a été déterminée successivement au profilomètre Dektak, puis par AFM en mode *« Détection de courant »,*
- des couches de 2,2',6,6'-tétraphényl-4,4'-dithiopyrannylidène de différentes épaisseurs, le composé de l'invention ayant été appliqué sur des substrats d'ITO par MO-CVD, à un régime d'évaporation stable de 0,1 Å/s. Les épaisseurs ont également été déterminées au profilomètre Dektak et par AFM en mode « *Détection de courant ».*

*b) Cartographie bidimensionnelle des propriétés électriques des substrats de l'invention*

Les films à base de 2,2',6,6'-tétraphényl-4,4'-dithiopyrannylidène, obtenus sous un régime d'évaporation stable de 0,1 Å/s, possèdent des morphologies similaires et sont composées de particules ayant un diamètre d'environ 200 nm (cf. ci-dessus).

La **Figure 6** représente un film à base de 2,2',6,6'-tétraphényl-4,4'-dithiopyrannylidène et un film à base de PEDOT : PSS capturées par AFM en mode *« Détection de courant »* pour une différence de potentiel de -600 mV. L'image associée à la couche de 2,2',6,6'-tétraphényl-4,4'-dithiopyrannylidène fait apparaître de nombreux contrastes en courant, correspondant aux chemins de conduction préférentiels des trous. Ces contrastes de courant apparaissent dès les faibles valeurs de polarisation, ce qui signifie que les résistances associées au volume de la couche organique sont faibles. Pour une même différence de potentiel de -600 mV, on observe que les chemins de conduction sont beaucoup moins nombreux et moins intenses dans le cas du PEDOT : PSS. La collection des trous est donc plus

importante dans le cas du film de l'invention.

*c) Caractérisation du régime d'injection des charges*

*Cas du $P_3HT$ (référence) :*

Des mesures locales de courant ont été réalisées sur une couche de $P_3HT$ et sont représentés sur le graphe de la **Figure 7**.

La courbe obtenue se divise en deux régions asymétriques : on y distingue une variation hyperbolique du courant pour des tensions négatives du substrat d'ITO et un courant très faible pour des tensions positives. Cette dépendance asymétrique et hyperbolique du courant indique un transport de charges dominé par une zone d'espace de charges défini selon la loi de Mott-Gurney :

$$J = 9/8\ \varepsilon_r\, \varepsilon_o\, \mu\ (V^2/L^3)$$

dans laquelle :

- J est la densité de charges mesurée, où J = I/S (I est le courant directement mesuré par le préamplificateur et S = 100 $nm^2$ est la surface de contact entre la pointe et le substrat, calculée à partir du modèle de Hertz),
- $\varepsilon_r$ est la constante diélectrique du polymère prise égale à 3,
- $\varepsilon_0$ est la permittivité relative du vide prise égale à $8{,}854187.10^{-12}\ m^{-3}.\ kg^{-1}.\ s^4.\ A^2$,
- $\mu$ représente la mobilité des charges,
- *V* est le potentiel appliqué (entre 2 V et -2 V), et
- *L* est l'épaisseur du film de $P_3HT$ (e = 47 nm).

Cette loi permet de calculer le flux maximum de charges qui peut s'écouler au travers d'une géométrie plane.

*Cas du 2,2',6,6'-tétraphényl-4,4'-dithiopyrannylidène :*

Des mesures locales de courant ont été réalisées par CS-AFM sur des substrats selon l'invention revêtus de films à base de 2,2',6,6'-tétraphényl-4,4'-dithiopyrannylidène, et sont comparées à celles obtenues pour la référence $P_3HT$ (cf. Figure 8). La courbe obtenue pour le film de l'invention est divisée en deux régions où le courant passe différemment. Ce comportement suggère que le film de l'invention est un film semi-conducteur de type p et confirme que son niveau énergétique HOMO se situe entre les travaux de sortie du Pt et du substrat d'ITO.

Les mobilités des charges positives calculées sont représentées sur la **Figure 9** et sont supérieures d'un facteur 10 à celles obtenues pour le $P_3HT$.

**E- <u>Application aux dispositifs OSC</u>**

Un dispositif OSC comprenant un substrat d'ITO revêtu d'un film selon l'invention (dispositif 1) a été comparé à :

- un dispositif OSC de référence comprenant uniquement un substrat d'ITO et une couche active photosensible (dispositif 2), et
- un dispositif OSC de référence comprenant un substrat d'ITO recouvert d'un polymère conducteur à base de PEDOT : PSS, puis d'une couche active photosensible (dispositif 3).

Ces trois dispositifs sont schématisés à la **Figure 10**.

<u>Fabrication du dispositif OSC de l'invention (dispositif 1) :</u>

Des substrats d'ITO (Sigma-Aldrich) ayant une résistivité d'environ 40 $\Omega.cm^{-2}$ sont décapés et lavés selon le protocole décrit ci-après.

Des substrats de 2,2 cm x 2,5 cm sont découpés avec un stylo à pointe diamantée, les particules de verre résiduelles enlevées par un flux d'air localisé sur les découpes, et les substrats obtenus lavés à l'eau savonneuse et sécher à l'air libre. Une piste conductrice est ensuite inscrite au centre des substrats par décapage de l'ITO (processus dit de *« etching »*) : après avoir été partiellement recouverts d'une bande de scotch, les substrats sont plongés dans

une solution comprenant 20 mL d'acide chlorhydrique concentré (à 47%), 20 mL d'eau distillée, et environ 2 g de chlorure de fer. Ce processus électrochimique permet d'enlever l'ITO non recouvert par le scotch, les substrats décapés étant ensuite lavés trois fois de suite à l'eau savonneuse et à l'eau distillée. Les résidus de scotch sont ensuite retirés. Après avoir été séché par un flux d'air, les substrats sont plongés dans deux bains de sonification, à température ambiante, pendant dix minutes : le premier bain étant réalisé dans l'acétone, et le second dans l'éthanol. Les substrats sont ensuite séchés par un flux d'azote, puis placés sous une lampe UV pour subir un décapage de 20 minutes à l'ozone : la lampe dégrade les molécules d'oxygène qui arrivent dans l'enceinte, l'ozone formé étant un puissant oxydant qui permet d'enlever les dernières contaminations carbonées issues de l'air ambiant. Les substrats traités sont ensuite sortis de l'enceinte et utilisés immédiatement.

Le composé de formule (I) est ensuite déposé sur les substrats d'ITO tout juste traité, ledit dépôt étant réalisé par voie sèche à une température d'environ 150°C, sous une pression de $10^{-6}$ mbar, pendant une durée de 3 heures.

Une couche active photosensible constituée d'un mélange en volume de $P_3$HT-PCBM (Sigma-Aldrich) (ratio en poids : 1/0,8), dans 1 mL de chlorobenzène est déposée à la tournette (« *spin coating* ») à une vitesse de 1000 tours/min pendant 20 secondes, puis à 1400 tours/min pendant 20 secondes, et enfin à 1000 tours/min pendant 10 secondes, de façon à obtenir une couche d'épaisseur de 110 nm. Un recuit à 120°C dans un four tubulaire sous atmosphère d'argon peut éventuellement être réalisé (voir ci-après). Le recuit permet d'optimiser la morphologie et la structure interne de la couche active, augmentant ainsi les performances photovoltaïques du dispositif OSC.

Du fluorure de lithium LiF (Sigma-Aldrich) est ensuite évaporé thermiquement sous un régime stable d'évaporation de 0,1 Å/s, jusqu'à obtention d'une couche ayant une épaisseur comprise entre 5 et 7 Å.

Des électrodes d'aluminium ayant une épaisseur de 80 nm sont ensuite déposées thermiquement sous un régime stable d'évaporation compris entre 1 et 2 Å/s et sous une pression de $10^{-6}$ mbar.

Fabrication des dispositifs OSC de référence (dispositifs 2 et 3) :

Les dispositifs 2 et 3 ont été préparés selon le même protocole que décrit précédemment (cf. dispositif 1).

Pour le dispositif 2, la couche active photosensible a été déposée directement sur un substrat d'ITO.

Pour le dispositif 3, un polymère conducteur PEDOT : PSS (Sigma-Aldrich) est déposé à la tournette (« *spin coating »)* à une vitesse de 2000 tours/min pendant 50 secondes, puis recuit pendant 30 minutes à 120°C dans un four tubulaire sous atmosphère d'argon, de façon à obtenir un film de 20 nm d'épaisseur.

Propriétés photovoltaïques comparées des dispositifs 1, 2 et 3, **sans recuit** de la couche active :

*a) Caractéristiques dans l'obscurité*

Les caractéristiques dans l'obscurité sont déterminées en posant un chiffon noir sur les dispositifs.

Dans l'obscurité, une cellule solaire suit le comportement d'une diode classique. Selon que la tension appliquée est supérieure ou inférieure à une tension seuil, la diode est respectivement « passante » ou « bloquante ». Le courant $I_d$ dans la diode suit une équation de type Schokley :

$$I_d = I_s (\exp(eV/nkT)-1)$$

où :

- $I_s$ est le courant de saturation sous polarisation inverse,
- e est la charge de l'électron,
- $V = V_{appliqué} - V_{bi}$, $V_{bi}$ étant le potentiel interne, et
- n est le facteur d'idéalité de la diode (0 < n < 1 où 1 est le cas idéal),
- k est la constante de Boltzmann, et
- T est la température.

Pour un potentiel infini, on peut calculer le coefficient d'idéalité de la diode selon l'équation suivante :

$$n = (eV/kT) (\ln(R))^{-1}$$

où R est le ratio de courant.

Les ratios de courant (+/- 1 V) et les résistances shunt obtenus pour les trois dispositifs sont récapitulées dans le Tableau III.

**Tableau III :**

| Dispositif | Ratio de courant | Rshunt (en $\Omega.cm^{-2}$) |
|---|---|---|
| **Dispositif 1** | $1{,}87.10^3$ | $2{,}5.10^5$ |
| **Dispositif 2** | $7{,}8.10^3$ | $1{,}43.10^6$ |
| **Dispositif 3** | $1{,}4.10^2$ | $10^5$ |

La résistance shunt est l'inverse de la pente de la caractéristique courant-tension au point de court-circuit (lorsque V = 0 V), et correspond aux courants de fuite présents dans la diode, ces derniers étant liés à la présence de recombinaisons de porteurs de charges dans la diode.

*b) Caractéristiques sous éclairement*

Les caractéristiques sous éclairement sont mesurées au moyen d'une lampe Oriel au xénon délivrant une puissance de 75 $mW.cm^{-2}$ au niveau de la diode (sous illumination). Un filtre AMG 1.5 (Air Mass Global 1.5) est placé entre la lampe et la cellule, ce filtre permettant de reproduire le spectre de lumière blanche du spectre solaire.

Les résultats obtenus sont rassemblés dans le tableau suivant :

**Tableau IV :**

| Dispositif | Vmax (mV) | Imax ($\mu$A) | Isc ($\mu$A) | Facteur de Forme (FF) | Courant photogénéré Jsc ($mA.cm^{-2}$) |
|---|---|---|---|---|---|
| **Dispositif 1** | 260,7 | 502,5 | 801,76 | 0,3948 | 5,074 |
| **Dispositif 2** | 311,6 | 426,81 | 706,52 | 0,3677 | 4,07 |
| **Dispositif 3** | 443,3 | 308,06 | 524,23 | 0,3940 | 4,68 |

En dessinant la caractéristique courant tension d'une cellule dans l'obscurité et sous éclairement, il est possible d'évaluer les performances et le comportement électrique des diodes. On peut ainsi définir les paramètres suivants (cf. **Figure 11**) :

- le courant de court-circuit Isc obtenu pour une tension nulle. Ce courant est proportionnel à l'éclairement incident (Jsc = Isc/S, où S est la surface active de l'électrode),
- la tension en circuit ouvert Voc mesurée pour un courant nul,
- Imax et Vmax sont les coordonnées I-V (Pmax = Imax x Vmax), représentés par le rectangle gris foncé sur la **Figure 11**,
- le Facteur de Forme (FF) est égal à : FF = (Vmax x Imax) / (Voc x Isc)

Les courbes I-V obtenues pour les trois dispositifs sont représentés sur la **Figure 12**.

On constate que le dispositif 1 de l'invention présente de meilleures performances que celles obtenues pour les dispositifs 2 et 3, et notamment :

> ➢ le courant photogénéré Jsc est supérieur de plus de 20% à celui mesuré pour le dispositif 2 (plus de 1 $mA.cm^{-2}$ d'augmentation). Ce courant est également supérieur à celui obtenu pour le dispositif 3, ce qui montre que les trous sont collectés de manière plus efficace dans le dispositif de l'invention, et
> ➢ le facteur de forme FF est également légèrement supérieur pour le dispositif de l'invention.

*c) Détermination expérimentale du photocourant*

Quand un semi-conducteur est exposé à des photons d'énergie supérieure à celle de son gap optique, des porteurs de charges sont créés. L'existence d'un potentiel intrinsèque au matériau ou l'application d'un potentiel externe peut provoquer la séparation des charges et produire un photocourant dans un circuit externe.

Expérimentalement, le photocourant se détermine des courbes de réponse en I-V dans l'obscurité et sous éclairement, grâce à la différence ($J_L$ - $J_D$), où $J_L$ est la densité de courant obtenue sous éclairement et $J_D$ est la densité

de courant obtenue dans l'obscurité.

L'évolution du photocourant $J_{ph}$ en fonction du potentiel effectivement appliqué au dispositif ($V_o$-V), où $V_o$ est le potentiel de compensation déterminé sur la courbe de réponse I-V dans l'obscurité (et qui donne une mesure directe du potentiel intrinsèque $V_{bi}$) et V est le potentiel appliqué aux électrodes, permet de discriminer les régions où le photocourant est dominé par des phénomènes de recombinaison (monomoléculaire ou bimoléculaires), ou par des effets liés à un régime d'espace de charges, ou par des effets compétitifs entre courant de dérive et courant de diffusion.

Les courbes de réponse du photocourant en fonction du potentiel effectif dans le dispositif ($V_o$-V) sont représentées sur la **Figure 13**.

Pour des valeurs de ($V_o$-V) inférieures à 0,1 V, on observe un régime linéaire pour les dispositifs 2 et 3, et une légère déviation pour la courbe du dispositif 1 de l'invention. Ce phénomène s'explique par une plus grande participation du courant de dérive par rapport au courant de diffusion, pour de faibles tensions. Pour des valeurs de ($V_o$-V) supérieures à 0,1 V, on constate que le photocourant suit une loi en $V^{1/2}$, preuve de l'existence d'un régime de type SCLC (Space Charge Limited Current) ou de fortes recombinaisons monomoléculaires. Il est à noter qu'un régime de type SCLC se manifeste lorsqu'il se produit une accumulation de charges à l'une des électrodes du dispositif.

La dépendance du photocourant à l'intensité lumineuse incidente est également déterminée. Pour des valeurs de ($V_o$-V) choisies, on caractérise les valeurs des coefficients $\alpha$ de la loi de puissance $J = P^{\alpha}$.

<u>**Tableau V :**</u>

| **Dispositif** | **Coefficient $\alpha$** |
|---|---|
| **Dispositif 1** | 0,86 |
| **Dispositif 2** | 0,77 |
| **Dispositif 3** | 0,79 |

Les dispositifs 2 et 3 présentent des coefficients proches de 0,75. Ces valeurs, proches du coefficient théorique d'un régime de type SCLC, suggèrent que le transport au sein de la couche active de ces dispositifs est non équilibré et limité par la mobilité des trous de la couche active. Le transport au sein des dispositifs 2 et 3 est donc limité par une accumulation des trous à proximité de l'interface anodique.

Au contraire, la valeur du coefficient $\alpha$ déterminée pour le dispositif 1 de l'invention est supérieure et témoigne d'un régime plus équilibré, influant notamment sur l'équilibre du transport des charges de $P_3HT$. Le substrat de l'invention permet donc de réduire l'accumulation des charges à l'électrode, grâce aux nombreux chemins de conduction observés et aux niveaux électroniques étagés entre le substrat et la couche active photosensible.

<u>Influence de l'épaisseur et de la morphologie des films de l'invention sur des systèmes non recuits :</u>

Trois dispositifs A, B et C comprenant des substrats revêtus de films à base de 2,2',6,6'-tétraphényl-4,4'-dithiopyrannylidène et ayant des morphologies et épaisseurs différentes ont été évalués.

<u>**Tableau VI :**</u>

| **Dispositif** | **Régime d'évaporation des films (Å/s)** | **Epaisseur de la couche (nm)** |
|---|---|---|
| **Dispositif A** | 0,1 | 18 |
| **Dispositif B** | 0,1 | 45 |
| **Dispositif C** | >2 | 37* |
| L'épaisseur indiquée pour le dispositif C est une moyenne car le film est inhomogène du fait d'un régime d'évaporation instable. | | |

*a) Caractéristiques dans l'obscurité*

Les ratios de courant (+/- 2V) et les résistances shunt et de séries ont été déterminées et sont récapitulées dans le Tableau VII.

**Tableau VII :**

| Dispositif | Ratio de courant | Rshunt ($\Omega.cm^{-2}$) | Rséries ($\Omega.cm^{-2}$) |
|---|---|---|---|
| **Dispositif A** | $1,2.10^3$ | $2,5.10^5$ | 277,8 |
| **Dispositif B** | 90,9 | $5.10^4$ | $3,33.10^3$ |
| **Dispositif C** | 126,4 | $3,33.10^4$ | $2,5.10^4$ |

La résistance série est l'inverse de la pente de la caractéristique courant-tension obtenue pour une tension V supérieure à Voc.

Les performances des diodes dans l'obscurité dépendent très significativement des épaisseurs et des morphologies des épaisseurs des films.

La diode la plus efficace est celle qui présente l'épaisseur la moins importante, l'interface à base de 2,2',6,6'-tétraphényl-4,4'-dithiopyrannylidène devenant critique pour des épaisseurs trop importantes.

Les valeurs des résistances shunt observées pour les dispositifs B et C sont 10 fois inférieures à celle du dispositif A. Les valeurs des résistances séries sont, quant à elles, supérieures, témoignant de la diminution de la qualité des interfaces.

*b) Caractéristiques sous éclairement*

Les différents paramètres photovoltaïques obtenus sous éclairement AMG 1.5 sont rassemblés dans le tableau VIII suivant :

**Tableau VIII :**

| Dispositif | Vmax (mV) | Imax ($\mu$A) | Tension en circuit ouvert Voc (mV) | Isc ($\mu$A) | Facteur de Forme (FF) | Courant photogénéré Jsc ($A.cm^{-2}$) | Rendement d'efficacité globale PCE (%) |
|---|---|---|---|---|---|---|---|
| **Dispositif A** | 260,7 | 502,5 | 413,9 | 801,76 | 0,3948 | 5,074 | 1,11 |
| **Dispositif B** | 133,7 | 202,43 | 251,1 | 379,45 | 0,2841 | 3,098 | 0,22 |
| **Dispositif C** | 80,7 | 62,742 | 149,3 | 125,34 | 0,2706 | 1,066 | 0,06 |

Les courbes de réponse I-V sont représentées sur la **Figure 14** :

➢ La courbe correspondant au film hétérogène (dispositif C) présente un facteur de forme inversé (forme concave dite « S-curve »). Cette courbe en forme de S est généralement associée à la présence d'une interface de qualité médiocre au sein du dispositif, due à une évaporation trop rapide ou à une oxydation de l'électrode. Ce facteur de forme inversé suggère que l'interface n'est pas optimisée, cette dernière pouvant bloquer les charges libres collectées à l'ITO et ralentir le transfert de charges entre la couche active photosensible et le substrat de l'invention.

➢ La courbe correspondant au film ayant une épaisseur de 45 nm (dispositif B) présente un facteur de forme convexe assez faible. Une comparaison des courbes des dispositifs A et B suggère qu'une partie de la lumière incidente est absorbée par le substrat de l'invention, le nombre de charges photogénérés diminuant avec l'augmentation de l'épaisseur du film et expliquant les faibles valeurs de Voc et de Jsc obtenues pour le dispositif B (par rapport au dispositif A).

Les résistances shunt et de séries ont également été déterminées et confirment les tendances observées sur les courbes I-V (cf. Tableau IX) :

**Tableau IX :**

| Dispositif | Rshunt ($\Omega.cm^{-2}$) | Rséries ($\Omega.cm^{-2}$) |
|---|---|---|
| **Dispositif A** | 232 | 26,38 |

(suite)

| Dispositif | Rshunt (Ω.$cm^{-2}$) | Rséries (Ω.$cm^{-2}$) |
|---|---|---|
| **Dispositif B** | 137 | 59,9 |
| **Dispositif C** | 83,3 | 98,04 |

Propriétés photovoltaïques comparées des dispositifs 1, 2 et 3, **avec recuit** de la couche active :

Les dispositifs étudiés correspondent aux dispositifs 1, 2 et 3 décrits précédemment, la seule différence reposant sur le recuit de la couche active photosensible.

La couche de PEDOT : PSS est de 20 nm et l'épaisseur du film de l'invention est de 14 nm.

Les recuits ont été réalisés avant l'évaporation du fluorure de lithium (LiF) et de l'aluminium, en chauffant les dispositifs dans un four tubulaire à une température de 110°C pendant 10 minutes, sous une atmosphère inerte d'argon.

Il est à noter que le substrat de l'invention est totalement insensible à une telle température de chauffe (le point de fusion du 2,2',6,6'-tétraphényl-4,4'-dithiopyrannylidène étant de 328°C), aucun modification de la surface du substrat de l'invention n'ayant été observé par AFM en mode *« Détection de courant »* (pas de changement de l'occupation de la surface par le 2,2',6,6'-tétraphényl-4,4'-dithiopyrannylidène, ni de son caractère polycristallin, pas de variation de la taille des particules, ni de la rugosité).

*a) Caractéristigues dans l'obscurité*

Les ratios de courant (+/- 2V) et les résistances shunt et de séries ont été déterminées et sont récapitulées dans le Tableau X.

**Tableau X :**

| Dispositif | Ratio de courant | Rshunt (Ω.$cm^{-2}$) | Rséries (Ω.$cm^{-2}$) |
|---|---|---|---|
| **Dispositif 1** | 93,8 | $1{,}7.10^4$ | 14,5 |
| **Dispositif 2** | 373 | $5.10^4$ | 101,1 |
| **Dispositif 3** | 29,4 | $1.10^4$ | 29,9 |

Les valeurs des résistances séries et shunt obtenues sont approximativement du même ordre de grandeur pour les trois dispositifs. Les valeurs des résistances séries obtenues montrent que les substrats de l'invention (dispositif 1) forment des couches interfaciales de meilleure qualité que celles des dispositifs 2 et 3.

*b) Caractéristiques sous éclairement*

Les différents paramètres photovoltaïques obtenus sous éclairement AMG 1.5 sont rassemblés dans le tableau XI suivant :

**Tableau XI :**

| Dispositif | Vmax (mV) | Imax (μA) | Isc (μA) | Facteur de Forme (FF) | Courant photogénéré Jsc (A.$cm^{-2}$) | Rendement d'efficacité globale PCE (%) |
|---|---|---|---|---|---|---|
| **Dispositif 1** | 304,7 | 189,2 | 293,6 | 0,40 | 7,73 | 2,02 |
| **Dispositif 2** | 300,7 | 594,45 | 979,44 | 0,32 | 6,996 | 1,73 |
| **Dispositif 3** | 455,8 | 63,4 | 93,1 | 0,49 | 8,46 | 3,52 |

Les courbes de réponse I-V sont représentées sur la **Figure 15**.

On observe, pour le dispositif utilisant une couche interfaciale anodique selon l'invention, une amélioration

générale des performances photovoltaïques après recuit. Le facteur de forme du dispositif 1 est élevé et compris entre ceux des dispositifs 2 et 3 de référence. La densité de courant du dispositif 1 est également comprise entre celles des dispositifs 2 et 3.

*c) Détermination expérimentale du photocourant*

Afin de différencier les processus de recombinaisons intervenants dans la génération du photocourant, la dépendance du photocourant à l'intensité lumineuse incidente a été étudiée. Comme précédemment, les valeurs des coefficients $\alpha$ de la loi de puissance $J = P^{\alpha}$ ont été déterminées pour des valeurs ($V_o$-V) choisies.

**Tableau XII :**

| Dispositif | Coefficient $\alpha$ |
|---|---|
| **Dispositif 1** | 0,87 |
| **Dispositif 2** | 0,84 |
| **Dispositif 3** | 0,84 |

On constate que les coefficients $\alpha$ sont tous du même ordre de grandeur. Grâce au recuit de la couche active, les mobilités des charges libres sont équilibrées dans le volume du matériau, ce qui améliore le réseau de charges dans le matériau et réduit la forte accumulation des trous à proximité de l'interface anodique.

Le coefficient de puissance de la couche de l'invention (dispositif 1) est supérieur à ceux des dispositifs 2 et 3, mettant ainsi en évidence l'influence de la couche interfaciale à base de 2,2',6,6'-tétraphényl-4,4'-dithiopyrannylidène : les probabilités de recombinaison des charges dissociées sont affaiblies, les trous étant davantage collectés à l'anode.

Propriétés photovoltaïques d'un dispositif « hybride » et de systèmes de référence, **sans recuit** de la couche active :

Cette étude se base sur trois types de dispositifs dotés de couches interfaciales anodiques dites « hybrides », et a pour objectif de déterminer les paramètres influant sur la tension en circuit ouvert Voc.

A cet effet, trois dispositifs différents ont été étudiés :

- un dispositif X (dispositif « hybride ») comprenant un substrat d'ITO revêtue d'une première couche homogène de 2,2',6,6'-tétraphényl-4,4'-dithiopyrannylidène ayant une épaisseur de 15 nm, elle-même revêtue d'une couche de PEDOT: PSS de 5 nm d'épaisseur, puis d'une couche active photosensible,
- un dispositif Y comprenant un substrat d'ITO revêtue d'une couche de PEDOT : PSS ayant une épaisseur de 5 nm, puis d'une couche active photosensible,
- un dispositif Z comprenant un substrat d'ITO revêtue uniquement d'une couche active photosensible.

Les couches actives photosensibles sont à base de $P_3$HT-PCBM et ont été préparées comme décrit précédemment dans le paragraphe « Fabrication des dispositifs OSC de référence ». Les couches actives n'ont subies aucun recuit.

La caractérisation par AFM en mode *« Détection de courant »* des différents dispositifs a permis de mettre en évidence une évolution nette du recouvrement grâce au film à base de 2,2',6,6'-tétraphényl-4,4'-dithiopyrannylidène (cf. **Figure 16**). On distingue clairement sur ces images la participation des particules de 2,2',6,6'-tétraphényl-4,4'-dithiopyrannylidène sur la morphologie du réseau fibrillaire de la couche « hybride ».

*a) Caractéristiques dans l'obscurité*

Les ratios de courant (+/- 2V) et les résistances shunt et de séries ont été déterminées et sont récapitulées dans le Tableau XIII.

**Tableau XIII :**

| Dispositif | Ratio de courant | Rshunt ($\Omega.cm^{-2}$) | Rséries ($\Omega.cm^{-2}$) |
|---|---|---|---|
| **Dispositif X** | 795 | $2,5.10^5$ | 36,9 |
| **Dispositif Y** | 34,9 | $5.10^3$ | 117,6 |

(suite)

| Dispositif | Ratio de courant | Rshunt ($\Omega.cm^{-2}$) | Rséries ($\Omega.cm^{-2}$) |
|---|---|---|---|
| **Dispositif Z** | $7,8.10^3$ | $1,43.10^6$ | 294,1 |

Les phénomènes de recombinaison sont moins importants pour le dispositif de l'invention, témoignant d'un réseau de bonne qualité.

On remarque également que l'interface constituée uniquement de 2,2',6,6'-tétraphényl-4,4'-dithiopyrannylidène est de meilleure qualité que celles des dispositifs X, Y et Z (cf. résultats précédents).

*b) Caractéristiques sous éclairement*

Les différents paramètres photovoltaïques obtenus sous éclairement AMG 1.5 sont rassemblés dans le tableau XIV suivant :

**Tableau XIV :**

| Dispositif | Vmax (mV) | Imax ($\mu$A) | Voc (mV) | Isc ($\mu$A) | Facteur de Forme (FF) | Courant photogénéré Jsc ($A.cm^{-2}$) | Rendement d'efficacité globale PCE (%) |
|---|---|---|---|---|---|---|---|
| **Dispositif X** | 350,1 | 166,53 | 523 | 263,58 | 0,423 | 5,23 | 1,54 |
| **Dispositif Y** | 362,1 | 374,75 | 591,2 | 591,82 | 0,39 | 4,065 | 1,24 |
| **Dispositif Z** | 311,6 | 426,81 | 511,9 | 706,52 | 0,3677 | 4,07 | 1,02 |

On constate que le dispositif « hybride » à base de 2,2',6,6'-tétraphényl-4,4'-dithiopyrannylidène et de PEDOT : PSS est le plus performant des trois systèmes.

*c) Détermination expérimentale du photocourant*

Comme précédemment, les valeurs des coefficients $\alpha$ de la loi de puissance $J = P^{\alpha}$ ont été déterminées pour des valeurs ($V_o$-V) choisies.

**Tableau XV :**

| Dispositif | Coefficient $\alpha$ |
|---|---|
| **Dispositif X** | 0,88 |
| **Dispositif Y** | 0,78 |
| **Dispositif Z** | 0,77 |

Les dispositifs de référence suivent majoritairement un régime d'accumulation des charges de type SCLC (coefficients proches de 0,75), contrairement au dispositif de l'invention. Les recombinaisons de charges étant faibles, ces porteurs sont récoltés de manière efficace aux électrodes.

La couche interfaciale « hybride » comprenant du 2,2',6,6'-tétraphényl-4,4'-dithiopyrannylidène intervient dans le collecte des trous par sa mobilité intrinsèque élevée en trous, les chemins de conduction de la couche hybride étant attribuable à la présence du composé de formule (I) de l'invention.

Propriétés photovoltaïques d'un dispositif « hybride » et de systèmes de référence, **avec recuit** de la couche active :

Cette étude se base sur les trois dispositifs précédents, la seule différence reposant sur le recuit de la couche active photosensible. On nomme ces trois dispositifs X', Y' et Z' par analogie avec les dispositifs X, Y et Z.

*a) Caractéristiques dans l'obscurité*

Les résistances shunt et de séries ont été déterminées et sont récapitulées dans le Tableau XVI.

**Tableau XVI :**

| Dispositif | Rshunt ($\Omega.cm^{-2}$) | Rséries ($\Omega.cm^{-2}$) |
|---|---|---|
| **Dispositif X'** | $1.10^5$ | 172,4 |
| **Dispositif Y'** | $1.10^4$ | 29,9 |
| **Dispositif Z'** | $5.10^4$ | 101,1 |

Les caractéristiques des diodes sont quasiment similaires dans l'obscurité.

*b) Caractéristiques sous éclairement*

Les différents paramètres photovoltaïques obtenus sous éclairement AMG 1.5 sont rassemblés dans le tableau XVII suivant :

**Tableau XVII :**

| Dispositif | Vmax (mV) | Imax ($\mu$A) | Voc (mV) | Isc ($\mu$A) | Facteur de Forme (FF) | Courant photogénéré Jsc ($A.cm^{-2}$) | Rendement d'efficacité globale PCE (%) |
|---|---|---|---|---|---|---|---|
| **Dispositif X'** | 381,3 | 636,47 | 601,6 | 992,82 | 0,41 | 8,864 | 2,89 |
| **Dispositif Y'** | 455,8 | 63,34 | 637,6 | 93,05 | 0,49 | 8,46 | 3,5 |
| **Dispositif Z'** | 300,7 | 594,45 | 580 | 979,44 | 0,32 | 6,99 | 1,7 |

On observe, pour le dispositif faisant intervenir une interface « hybride », une amélioration des performances suite au recuit de la couche active photosensible.

La densité de courant à court-circuit du dispositif X' est supérieure à celles des dispositifs de référence Y' et Z', ce qui s'explique par une participation favorable de la couche à base de 2,2',6,6'-tétraphényl-4,4'-dithiopyrannylidène (barrière d'énergie abaissée à l'interface de la couche « hybride » et de la couche active photosensible, favorisant le transfert de charges).

*c) Détermination expérimentale du photocourant*

Comme précédemment, les valeurs des coefficients $\alpha$ de la loi de puissance $J = P^{\alpha}$ ont été déterminées pour des valeurs ($V_o$-V) choisies.

**Tableau XVIII :**

| Dispositif | Coefficient $\alpha$ |
|---|---|
| **Dispositif X'** | 0,93 |
| **Dispositif Y'** | 0,84 |
| **Dispositif Z'** | 0,84 |

L'évolution des coefficients $\alpha$ avec recuit suit l'évolution des coefficients $\alpha$ obtenus sans recuit. Le coefficient $\alpha$ très élevé obtenu pour le dispositif « hybride » X' correspond à un effet conjoint de la diminution des dissociations des paires géminées des excitons, ainsi qu'a une diminution des charges dissociées.

## Revendications

**1.** Substrat **caractérisé en ce qu'**il est revêtu d'un film comprenant au moins un composé de formule (I) suivante :

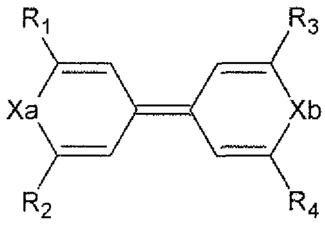

(I)

dans laquelle :

- Xa et Xb, identiques ou différents, sont choisis parmi les atomes N, P, O, S, Se ou Te,
- $R_1$, $R_2$, $R_3$ et $R_4$, identiques ou différents, représentent un groupement choisi parmi les cycles aryles ou hétéroaryles ayant 4 à 10 atomes de carbones, lesdits cycles aryles ou hétéroaryles pouvant éventuellement être substitués par un ou plusieurs atomes d'halogène, groupements hydroxyles -OH, alkyles ayant 1 à 30 atomes de carbone, alcoxy $-OC_nH_{2n+1}$ ou ester $-C(O)OC_nH_{2n+1}$, dans lesquels $0 \leq n \leq 16$,

le composé de formule (I) étant présent dans ledit film sous forme de particules ayant un diamètre moyen inférieur ou égal à 300 nm, de préférence inférieur ou égal à 250 nm, et encore plus préférentiellement inférieur ou égal à 200 nm.

**2.** Substrat selon la revendication 1 **caractérisé en ce que** les atomes Xa et Xb du composé de formule (I) sont identiques et choisis parmi les atomes O, S ou Se.

**3.** Substrat selon la revendication 2 **caractérisé en ce que** Xa = Xb = S.

**4.** Substrat selon l'une des revendications 1 à 3 **caractérisé en ce que** les cycles aryles ou hétéroaryles $R_1$, $R_2$, $R_3$ et $R_4$ du composé de formule (I) sont choisis parmi les cycles phényle, naphtyle, anthracyle, benzoxazolyle, thiophényle ou alcoxy-thiophényle, furyle, pyrrolyle, pyridyle, pyrazyle, pyrazolyle, pyridazyle, pyrimidyle, triazyle, imidazolyle, oxazolyle, indyle, indazolyle, quinolyle et quinoxalyle.

**5.** Substrat selon la revendication 4 **caractérisé en ce que** les cycles aryles ou hétéroaryles $R_1$, $R_2$, $R_3$ et $R_4$ du composé de formule (I) sont identiques et choisis parmi les cycles phényle, naphtyle ou alcoxy-thiophényle suivants :

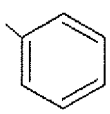 ; 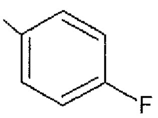 ; 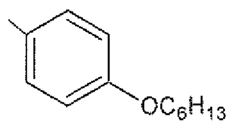 ; 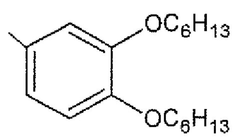 ;

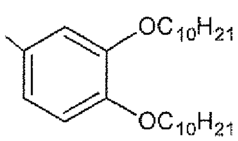 ; 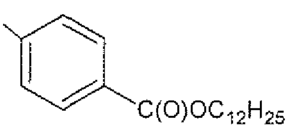 ; 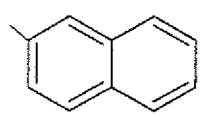 ; 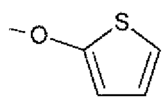

**6.** Substrat selon l'une des revendications 1 à 5 **caractérisé en ce qu'**il est revêtu d'un film comprenant au moins un composé de formule (I) choisi parmi les composés suivants ;

- Composé (1) :

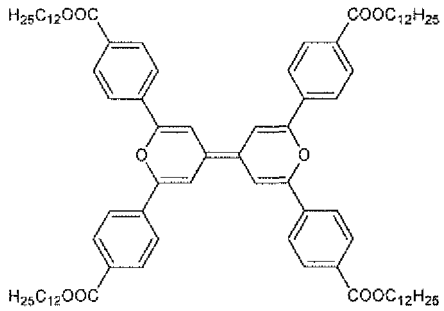

- Composé (2) :

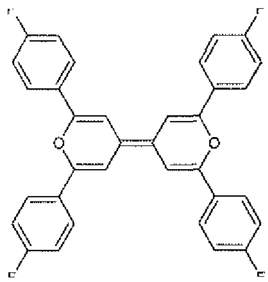

- Composé (3) :

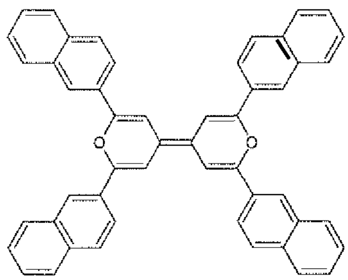

- Composé (4) :

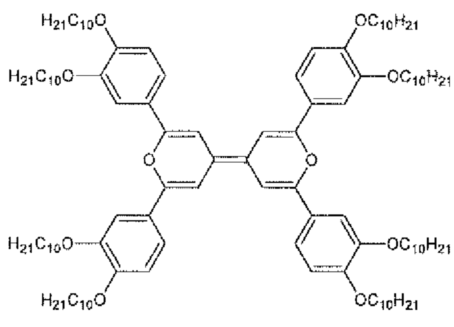

- Composé (5) :

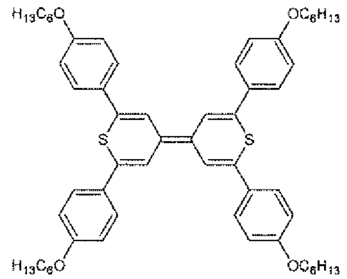

- Composé (6) :

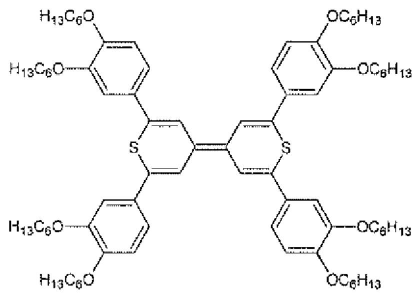

- Composé (7) :

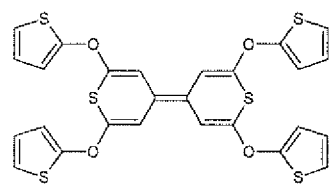

- Composé (8) :

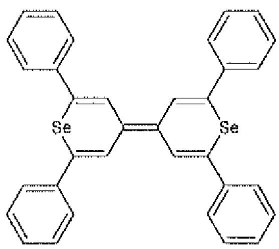

**7.** Substrat selon l'une des revendications 1 à 6 **caractérisé en ce que** ledit film a une épaisseur inférieure à 45 nm, de préférence inférieure ou égale à 30 nm, et encore plus préférentiellement inférieure ou égale à 15 nm.

**8.** Substrat selon l'une des revendications 1 à 7 **caractérisé en ce qu'**il est à base d'oxyde, et de préférence à base d'oxyde d'indium dopé à l'étain (ITO), d'oxyde de fer, d'oxyde d'aluminium ou d'oxyde de silicium.

**9.** Procédé de fabrication d'un substrat tel que défini selon l'une des revendications 1 à 8 **caractérisé en ce qu'**il comprend au moins une étape de dépôt par voie sèche d'un film comprenant un composé de formule (I) tel que défini selon l'une des revendications 1 à 8, ledit dépôt étant réalisé à une vitesse d'évaporation inférieure à 1 Å/s, de préférence inférieure ou égale à 0,4 Å/s, et encore plus préférentiellement inférieure ou égale à 0,1 Å/s.

**10.** Procédé selon la revendication 9 **caractérisé en ce que** le dépôt par voie sèche est réalisé par dépôt chimique en

phase vapeur CVD.

**11.** Procédé selon l'une des revendications 9 ou 10, **caractérisé en ce que** le dépôt par voie sèche est réalisé à une température de recuit comprise entre 80 et 120°C et sous une pression comprise entre $10^{-4}$ et $10^{-8}$ mbar, pendant une durée comprise entre 2 et 4 heures.

**12.** Utilisation d'un substrat selon l'une des revendications 1 à 8 comme couche interfaciale anodique dans des dispositifs électroniques tels que les diodes électroluminescentes organiques (OLED), les diodes électroluminescentes polymériques (PLED), les transistors organiques à effet de champ (OFET) et les cellules solaires organiques (OSC).

**13.** Utilisation selon la revendication 12 **caractérisée en ce que** la couche interfaciale anodique est une couche collectrice de trous.

**14.** Article fini choisi parmi les diodes électroluminescentes organiques (OLED), les diodes électroluminescentes polymériques (PLED), les transistors organiques à effet de champ (OFET) et les cellules solaires organiques (OSC), **caractérisé en ce qu'**il comprend au moins un substrat tel que défini selon l'une des revendications 1 à 8.

**15.** Cellule solaire organique (OSC) selon la revendication 14 **caractérisée en ce que** ledit substrat est à base d'oxyde d'indium dopé à l'étain (ITO).

**16.** Cellule solaire organique (OSC) selon l'une des revendications 14 ou 15 **caractérisée en ce qu'**elle comprend au moins un support de base (a) revêtu d'un substrat (b) tel que défini selon l'une des revendications 1 à 8, ledit substrat étant lui-même revêtu d'une couche active photosensible (c).

**17.** Cellule solaire organique (OSC) selon la revendication 16 **caractérisée en ce que** le support de base (a) est en verre, le substrat (b) est à base d'oxyde d'indium dopé à l'étain (ITO), et la couche active photosensible (c) est à base de $P_3HT$ : PCBM.

**18.** Cellule solaire organique (OSC) selon la revendication 17 **caractérisée en ce que** la couche active photosensible (c) est revêtue d'une couche (d) à base de fluorure de lithium (LiF), et ladite couche (d) est elle-même revêtue d'une couche électrolytique (e) à base d'aluminium, d'or, de calcium, de cuivre, de samarium, de platine, de palladium, de chrome, de cobalt ou d'iridium.

**19.** Procédé de fabrication d'une cellule solaire organique (OSC) telle que définie selon l'une des revendications 14 à 18 **caractérisé en ce qu'**il comprend au moins les étapes suivantes :

(i) le dépôt d'un substrat (b) tel que défini selon l'une des revendications 1 à 8 sur un support de base (a), puis
(i) le dépôt d'une couche active photosensible (c), ledit dépôt étant de préférence réalisé à la tournette,
(ii) éventuellement, le recuit de la couche active photosensible (c) dans un four tubulaire, à une température comprise entre 30 et 150°C, pendant une durée comprise entre 1 minute et 24 heures, sous une atmosphère inerte d'argon,
(iii) éventuellement, le dépôt d'une couche (d) à base de fluorure de lithium (LiF), ledit dépôt pouvant être réalisé par voie sèche à une vitesse d'évaporation inférieure à 1 Å/s, de préférence inférieure ou égale à 0,4 Å/s, et encore plus préférentiellement inférieure ou égale à 0,1 Å/s,
(iv) éventuellement, le dépôt d'une couche électrolytique (e), ledit dépôt pouvant être réalisé par voie sèche à une vitesse d'évaporation inférieure ou égale à 3 Å/s, et de préférence comprise entre 1 et 2 Å/s.

**20.** Composés de formule (I) suivante :

- Composé (1) :

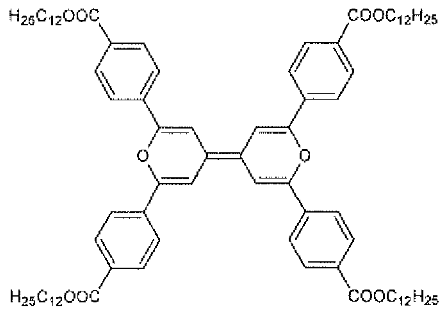

- Composé (3) :

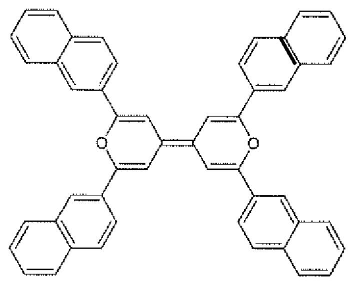

- Composé (5) :

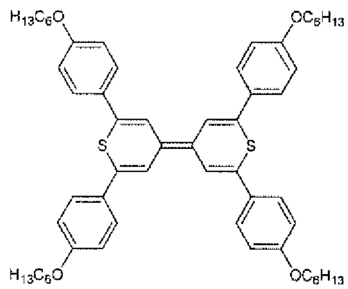

- Composé (6) :

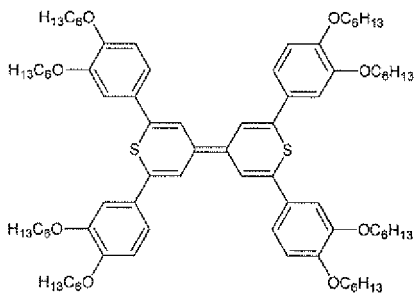

- Composé (7) :

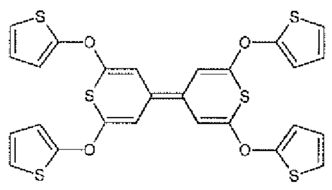

## Patentansprüche

1. Substrat, **dadurch gekennzeichnet, dass** es mit einem Film beschichtet ist, der wenigstens eine Verbindung der folgenden Formel (I) umfasst:

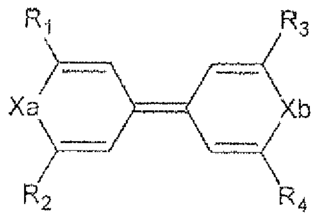

(I)

in der:

- Xa und Xb, die identisch oder unterschiedlich sind, aus den Atomen N, P, O, S, Se oder Te ausgewählt sind,
- $R_1$, $R_2$, $R_3$ und $R_4$, die identisch oder unterschiedlich sind, eine Gruppierung, ausgewählt aus Aryl- oder Heteroarylcyclen mit 4 bis 10 Kohlenstoffatomen, darstellen, wobei die Aryl- oder Heteroarylcyclen gegebenenfalls mit einem oder mehreren Halogenatomen, Hydroxylgruppen -OH, Alkylgruppen mit 1 bis 30 Kohlenstoffatomen, Alkoxygruppen $-OC_nH_{2n+1}$ oder Estergruppen $-C(O)OC_nH_{2n+1}$, in denen $0 \leq n \leq 16$, substituiert sein können,

wobei die Verbindung der Formel (I) in dem Film in Form von Partikeln, die einen mittleren Durchmesser von 300 nm oder weniger, vorzugsweise von 250 nm oder weniger und noch bevorzugter von 200 nm oder weniger haben, vorliegt.

2. Substrat gemäß Anspruch 1, **dadurch gekennzeichnet, dass** die Atome Xa und Xb der Verbindung der Formel (I) identisch sind und aus den Atomen O, S oder Se ausgewählt sind.

3. Substrat gemäß Anspruch 2, **dadurch gekennzeichnet, dass** Xa = Xb = S.

4. Substrat gemäß einem der Ansprüche 1 bis 3, **dadurch gekennzeichnet, dass** die Aryl- oder Heteroarylcyclen $R_1$, $R_2$, $R_3$ und $R_4$ der Verbindung der Formel (I) ausgewählt sind aus den Phenyl-, Naphthyl-, Anthracyl-, Benzoxazolyl-, Thiophenyl- oder Alkoxythiophenyl-, Furyl-, Pyrrolyl-, Pyridyl-, Pyrazyl-, Pyrazolyl-, Pyridazyl-, Pyrimidyl-, Triazyl-, Imidazolyl-, Oxazolyl-, Indyl-, Indazolyl-, Chinolyl- und Chinoxalyl-Cyclen.

5. Substrat gemäß Anspruch 4, **dadurch gekennzeichnet, dass** die Aryl- oder Heteroarylcyclen $R_1$, $R_2$, $R_3$ und $R_4$ der Verbindung der Formel (I) identisch sind und ausgewählt sind aus den folgenden Phenyl-, Naphthyl- oder Alkoxythiophenylcyclen:

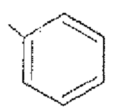 ; 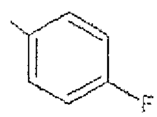 ; 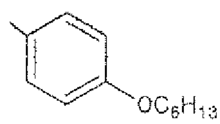 ; 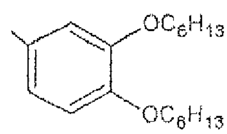 ;

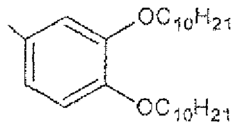 ; 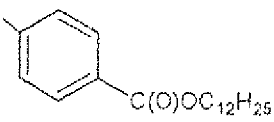 ; 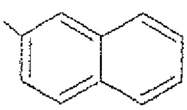 ; 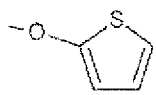

**6.** Substrat gemäß einem der Ansprüche 1 bis 5, **dadurch gekennzeichnet, dass** es mit einem Film beschichtet ist, der wenigstens eine Verbindung der Formel (I) umfasst, die aus den folgenden Verbindungen ausgewählt ist:

- Verbindung (1):

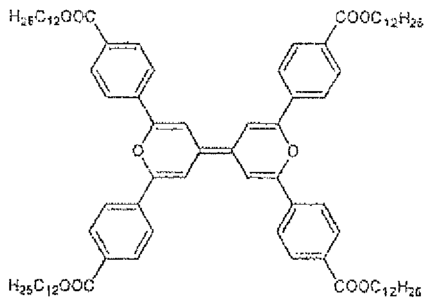

- Verbindung (2):

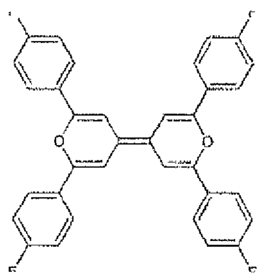

- Verbindung (3):

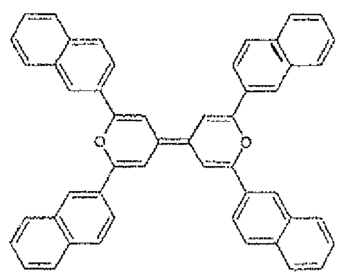

- Verbindung (4) :

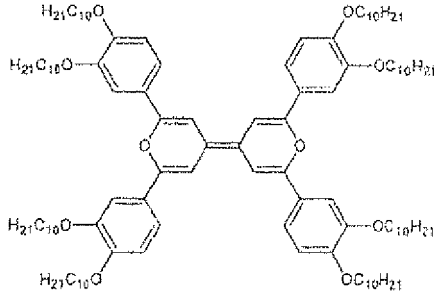

- Verbindung (5):

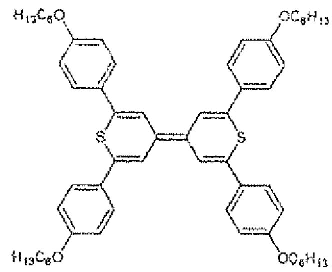

- Verbindung (6) :

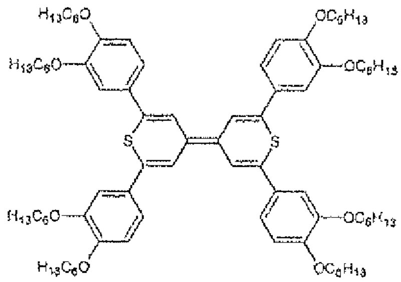

- Verbindung (7):

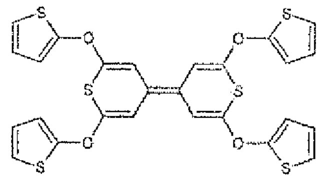

- Verbindung (8):

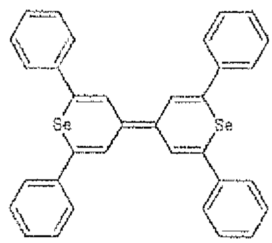

7. Substrat gemäß einem der Ansprüche 1 bis 6, **dadurch gekennzeichnet, dass** der Film eine Dicke von weniger als 45 nm, vorzugsweise von 30 nm oder weniger und noch bevorzugter von 15 nm oder weniger hat.

8. Substrat gemäß einem der Ansprüche 1 bis 7, **dadurch gekennzeichnet, dass** es auf Oxidbasis, und vorzugsweise auf der Basis von Zinn-dotiertem Indiumoxid (ITO), von Eisenoxid, von Aluminiumoxid oder von Siliciumoxid ist.

9. Verfahren zur Herstellung eines Substrats wie das gemäß einem der Ansprüche 1 bis 8 definierte, **dadurch gekennzeichnet, dass** es wenigstens eine Stufe der Abscheidung auf trockenem Weg eines Films, der eine Verbindung der Formel (I), wie sie in einem der Ansprüche 1 bis 8 definiert ist, umfasst, wobei die Abscheidung mit einer Verdampfungsgeschwindigkeit von unter 1 Å/s, vorzugsweise von 0,4 Å/s oder darunter und noch bevorzugter von 0,1 Å/s oder darunter durchgeführt wird.

**10.** Verfahren gemäß Anspruch 9, **dadurch gekennzeichnet, dass** die Abscheidung auf trockenem Weg durch chemische Dampfphasenabscheidung CVD durchgeführt wird.

**11.** Verfahren gemäß einem der Ansprüche 9 oder 10, **dadurch gekennzeichnet, dass** die Abscheidung auf trockenem Weg bei einer Wärmebehandlungstemperatur zwischen 80 und 120 °C und unter einem Druck zwischen $10^{-4}$ und $10^{-8}$ mbar während einer Zeitdauer, die zwischen 2 und 4 Stunden liegt, durchgeführt wird.

**12.** Verwendung eines Substrats gemäß einem der Ansprüche 1 bis 8 als anodische Grenzflächenschicht in elektronischen Vorrichtungen, zum Beispiel organisch elektrolumineszente Dioden (OLED), polymere elektrolumineszente Dioden (PLED), organische Feldeffekttransistoren (OFET) und organische Solarzellen (OSC).

**13.** Verwendung gemäß Anspruch 12, **dadurch gekennzeichnet, dass** die anodische Grenzflächenschicht eine Lochsammelschicht ist.

**14.** Endgegenstand, ausgewählt aus organischen elektrolumineszenten Dioden (OLED), polymeren elektrolumineszenten Dioden (PLED), organischen Feldeffekttransistoren (OFET) und organischen Solarzellen (OSC), **dadurch gekennzeichnet, dass** er wenigstens ein Substrat, wie es in einem der Ansprüche 1 bis 8 definiert ist, umfasst.

**15.** Organische Solarzelle (OSC) gemäß Anspruch 14, **dadurch gekennzeichnet, dass** das Substrat auf Basis von Zinn-dotiertem Indiumoxid (ITO) ist.

**16.** Organische Solarzelle (OSC) gemäß einem der Ansprüche 14 oder 15, **dadurch gekennzeichnet, dass** sie wenigstens einen Basisträger (a) beschichtet mit einem Substrat (b), wie es gemäß einem der Ansprüche 1 bis 8 definiert ist, umfasst, wobei das Substrat selbst mit einer aktiven lichtempfindlichen Schicht (c) beschichtet ist.

**17.** Organische Solarzelle (OSC) gemäß Anspruch 16, **dadurch gekennzeichnet, dass** der Basisträger (a) aus Glas ist, das Substrat (b) auf Basis von Zinn-dotiertem Indiumoxid (ITO) ist und die aktive lichtempfindliche Schicht (c) auf der Basis von $P_3HT$ : PCBM ist.

**18.** Organische Solarzelle (OSC) gemäß Anspruch 17, **dadurch gekennzeichnet, dass** die aktive lichtempfindliche Schicht (c) mit einer Schicht (d) auf der Basis von Lithiumfluorid (LiF) beschichtet ist und die Schicht (d) selbst mit einer Elektrolytschicht (e) auf der Basis von Aluminium, Gold, Calcium, Kupfer, Samarium, Platin, Palladium, Chrom, Kobalt oder Iridium beschichtet ist.

**19.** Verfahren zur Herstellung einer organischen Solarzelle (OSC), wie sie in einem der Ansprüche 14 bis 18 definiert ist, **dadurch gekennzeichnet, dass** es wenigstens die folgenden Stufen umfasst:

(i) die Abscheidung eines Substrates (b), wie es gemäß einem der Ansprüche 1 bis 8 definiert ist, auf einem Basisträger (a), dann
(i) Abscheidung einer aktiven lichtempfindlichen Schicht (c), wobei die Abscheidung vorzugsweise in einem Schleuderapparat durchgeführt wird,
(ii) gegebenenfalls Hitzbehandlung der aktiven lichtempfindlichen Schicht (c) in einem Rohrofen bei einer Temperatur zwischen 30 und 150°C während einer Zeit, die zwischen 1 Minute und 24 Stunden liegt, unter einer inerten Argonatmosphäre,
(iii) gegebenenfalls Abscheidung einer Schicht (d) auf der Basis von Lithiumfluorid (LiF), wobei die Abscheidung auf trockenem Weg bei einer Verdampfungsgeschwindigkeit von unter 1 Å/s, vorzugsweise 0,4 Å/s oder weniger und noch bevorzugter 0,1 Å/s oder darunter durchgeführt wird,
(iv) gegebenenfalls Abscheidung einer Elektrolytschicht (e), wobei die Abscheidung auf trockenem Weg bei einer Verdampfungsgeschwindigkeit von 3 Å/s oder darunter und vorzugsweise von zwischen 1 und 2 Å/s durchgeführt wird.

**20.** Verbindungen der folgenden Formel (I):

- Verbindung (1):

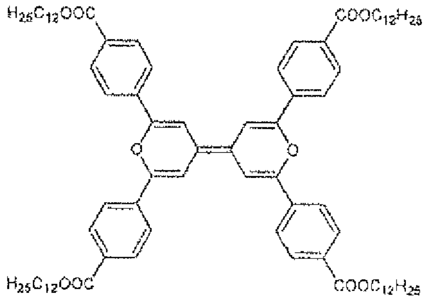

- Verbindung (3):

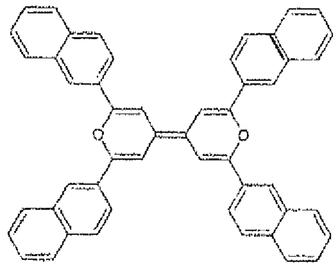

- Verbindung (5):

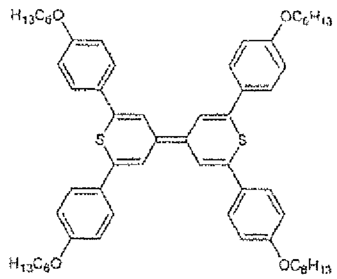

- Verbindung (6):

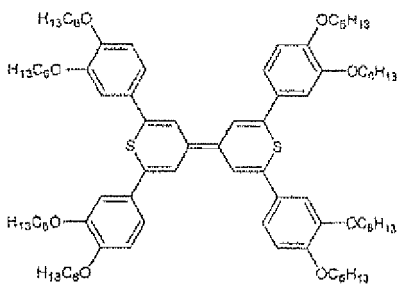

- Verbindung (7):

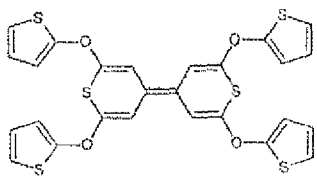

## Claims

1. Substrate, **characterised in that** it is coated with a film comprising at least one compound of the following formula (I):

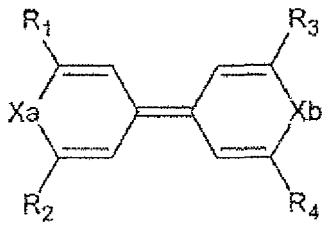

(I)

wherein:

- Xa and Xb, which may be identical or different, are chosen from the atoms N, P, O, S, Se or Te,
- $R_1$, $R_2$, $R_3$ and $R_4$, which may be identical or different, represent a group chosen from aryl or heteroaryl rings having from 4 to 10 carbon atoms, said aryl or heteroaryl rings optionally being substituted by one or more halogen atoms, hydroxyl groups -OH, alkyl groups having from 1 to 30 carbon atoms, alkoxy groups $-OC_nH_{2n+1}$ or ester groups $-C(O)OC_nH_{2n+1}$, wherein $0 \leq n \leq 16$,

the compound of formula (I) being present in said film in the form of particles having a mean diameter of less than or equal to 300 nm, preferably less than or equal to 250 nm, and more preferably less than or equal to 200 nm.

2. Substrate according to claim 1, **characterised in that** the atoms Xa and Xb of the compound of formula (I) are identical and chosen from the atoms O, S or Se.

3. Substrate according to claim 2, **characterised in that** Xa = Xb = S.

4. Substrate according to any one of claims 1 to 3, **characterised in that** the aryl or heteroaryl rings $R_1$, $R_2$, $R_3$ and $R_4$ of the compound of formula (I) are chosen from the rings phenyl, naphthyl, anthracyl, benzoxazolyl, thiophenyl or alkoxy-thiophenyl, furyl, pyrrolyl, pyridyl, pyrazyl, pyrazolyl, pyridazyl, pyrimidyl, triazyl, imidazolyl, oxazolyl, indyl, indazolyl, quinolyl and quinoxalyl.

5. Substrate according to claim 4, **characterised in that** the aryl or heteroaryl rings $R_1$, $R_2$, $R_3$ and $R_4$ of the compound of formula (I) are identical and chosen from the following phenyl, naphthyl or alkoxy-thiophenyl rings:

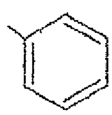 ; 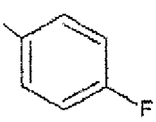 ; 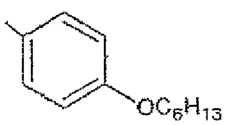 ; 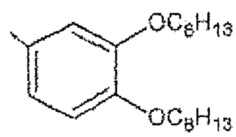 ;

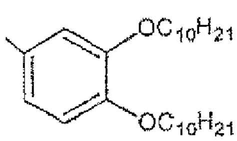 ; 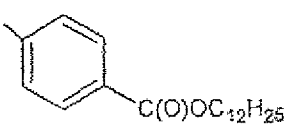 ; 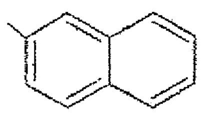 ; 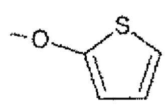

6. Substrate according to any one of claims 1 to 5, **characterised in that** it is coated with a film comprising at least one compound of formula (I) chosen from the following compounds:

- Compound (1):

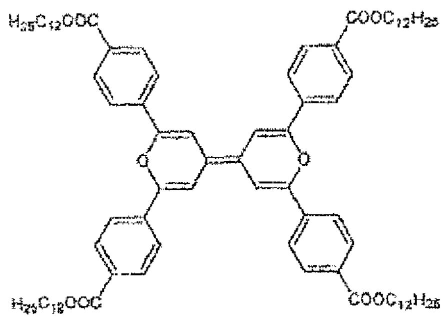

- Compound (2):

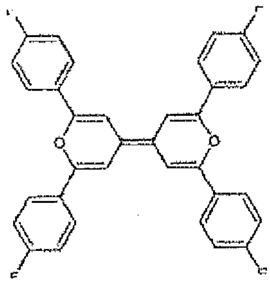

- Compound (3):

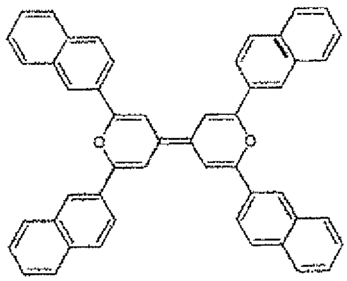

- Compound (4):

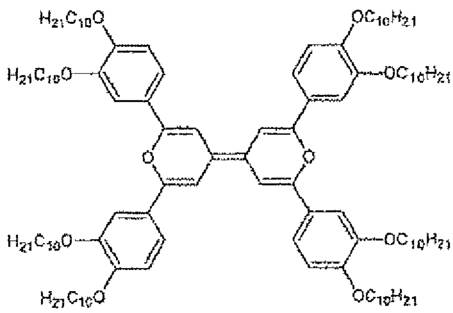

- Compound (5):

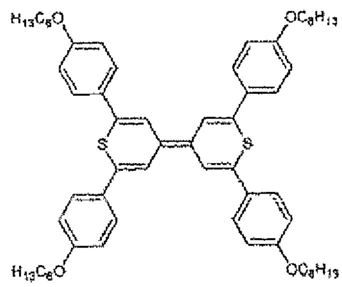

- Compound (6):

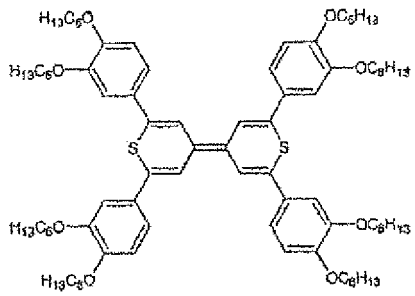

- Compound (7):

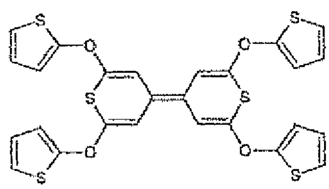

- Compound (8):

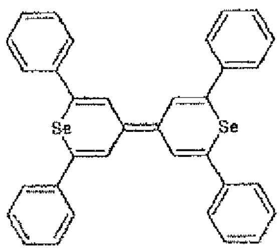

7. Substrate according to any one of claims 1 to 6, **characterised in that** said film has a thickness of less than 45 nm, preferably less than or equal to 30 nm, and more preferably less than or equal to 15 nm.

8. Substrate according to any one of claims 1 to 7, **characterised in that** it is based on oxide, preferably based on indium tin oxide (ITO), iron oxide, aluminium oxide or silicon oxide.

9. Process for the production of a substrate as defined according to any one of claims 1 to 8, **characterised in that** it comprises at least one step of dry deposition of a film comprising a compound of formula (I) as defined according to any one of claims 1 to 8, said deposition being carried out at a rate of evaporation of less than 1 A/s, preferably less than or equal to 0.4 Å/s, and more preferably less than or equal to 0.1 Å/s.

10. Process according to claim 9, **characterised in that** the dry deposition is carried out by chemical vapour deposition

CVD.

**11.** Process according to either claim 9 or claim 10, **characterised in that** the dry deposition is carried out at an annealing temperature of from 80 to 120°C and under a pressure of from $10^{-4}$ to $10^{-8}$ mbar, for a period of from 2 to 4 hours.

**12.** Use of a substrate according to any one of claims 1 to 8 as an anode interfacial layer in electronic devices such as organic light emitting diodes (OLED), polymer light emitting diodes (PLED), organic field effect transistors (OFET) and organic solar cells (OSC).

**13.** Use according to claim 12, **characterised in that** the anode interfacial layer is a hole collecting layer.

**14.** Finished article chosen from organic light emitting diodes (OLED), polymer light emitting diodes (PLED), organic field effect transistors (OFET) and organic solar cells (OSC), **characterised in that** it comprises at least one substrate as defined according to any one of claims 1 to 8.

**15.** Organic solar cell (OSC) according to claim 14, **characterised in that** said substrate is based on indium tin oxide (ITO).

**16.** Organic solar cell (OSC) according to either claim 14 or claim 15, **characterised in that** it comprises at least one base support (a) coated with a substrate (b) as defined according to any ane of claims 1 to 8, said substrate itself being coated with an active photosensitive layer (c).

**17.** Organic solar cell (OSC) according to claim 16, **characterised in that** the base support (a) is glass, the substrate (b) is based on indium tin oxide (ITO) and the active photosensitive layer (c) is based on $P_3HT$ : PCBM.

**18.** Organic solar cell (OSC) according to claim 17, **characterised in that** the active photosensitive layer (c) is coated with a layer (d) based on lithium fluoride (LiF) and said layer (d) is itself coated with an electrolytic layer (e) based on aluminium, gold, calcium, copper, samarium, platinum, palladium, chromium, cobalt or iridium.

**19.** Process for the production of an organic solar cell (OSC) as defined according to any ane of claims 14 to 18, **characterised in that** it comprises at least the following steps:

(i) deposition of a substrate (b) as defined according to any one of claims 1 to 8 on a base support (a), then
(i) deposition of an active photosensitive layer (c), said deposition preferably being carried out by spin coating,
(ii) optionally, annealing of the active photosensitive layer (c) in a tube furnace at a temperature of from 30 to 150°C for a period of from 1 minute to 24 hours, under an inert argon atmosphere,
(iii) optionally, deposition of a layer (d) based on lithium fluoride (LiF), it being possible for said deposition to be carried out by the dry method at a rate of evaporation of less than 1 Å/s, preferably less than or equal to 0.4 Å/s, and more preferably less than or equal to 0.1 Å/s,
(iv) optionally, deposition of an electrolytic layer (e), it being possible for said deposition to be carried out by the dry method at a rate of evaporation of less than or equal to 3 Å/s, and preferably from 1 to 2 Å/s.

**20.** Compounds of the following formula (I):

- Compound (1):

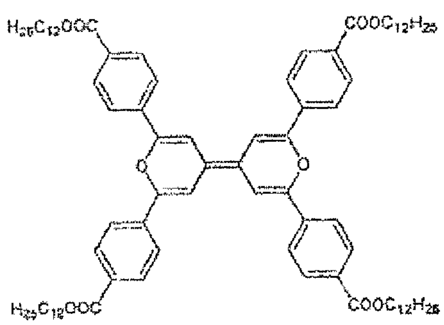

- Compound (3):

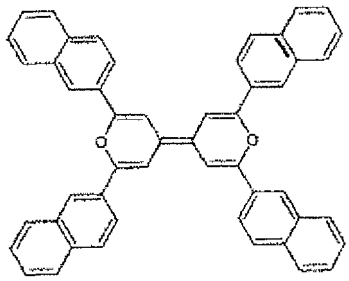

- Compound (5):

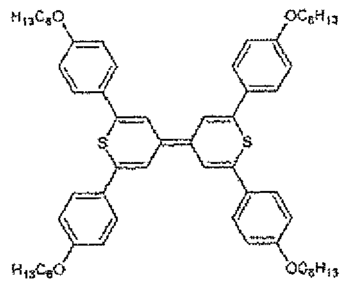

- Compound (6):

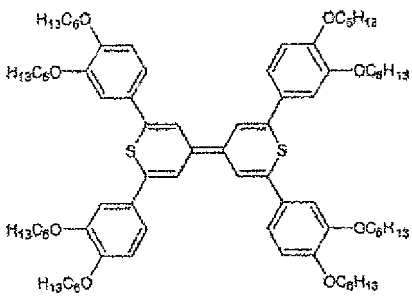

- Compound (7):

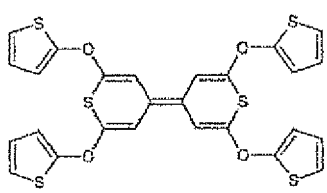

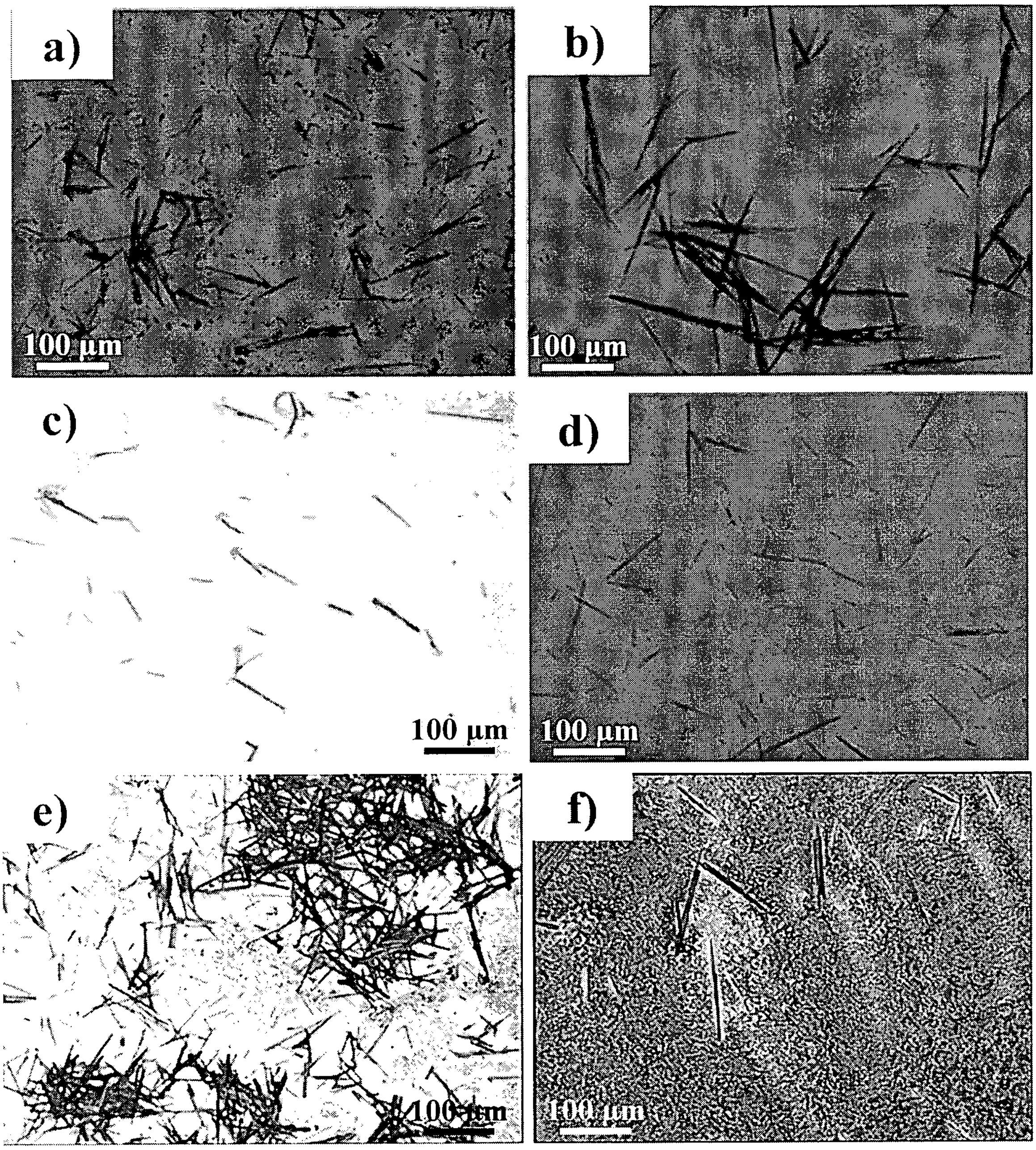


FIGURE 1

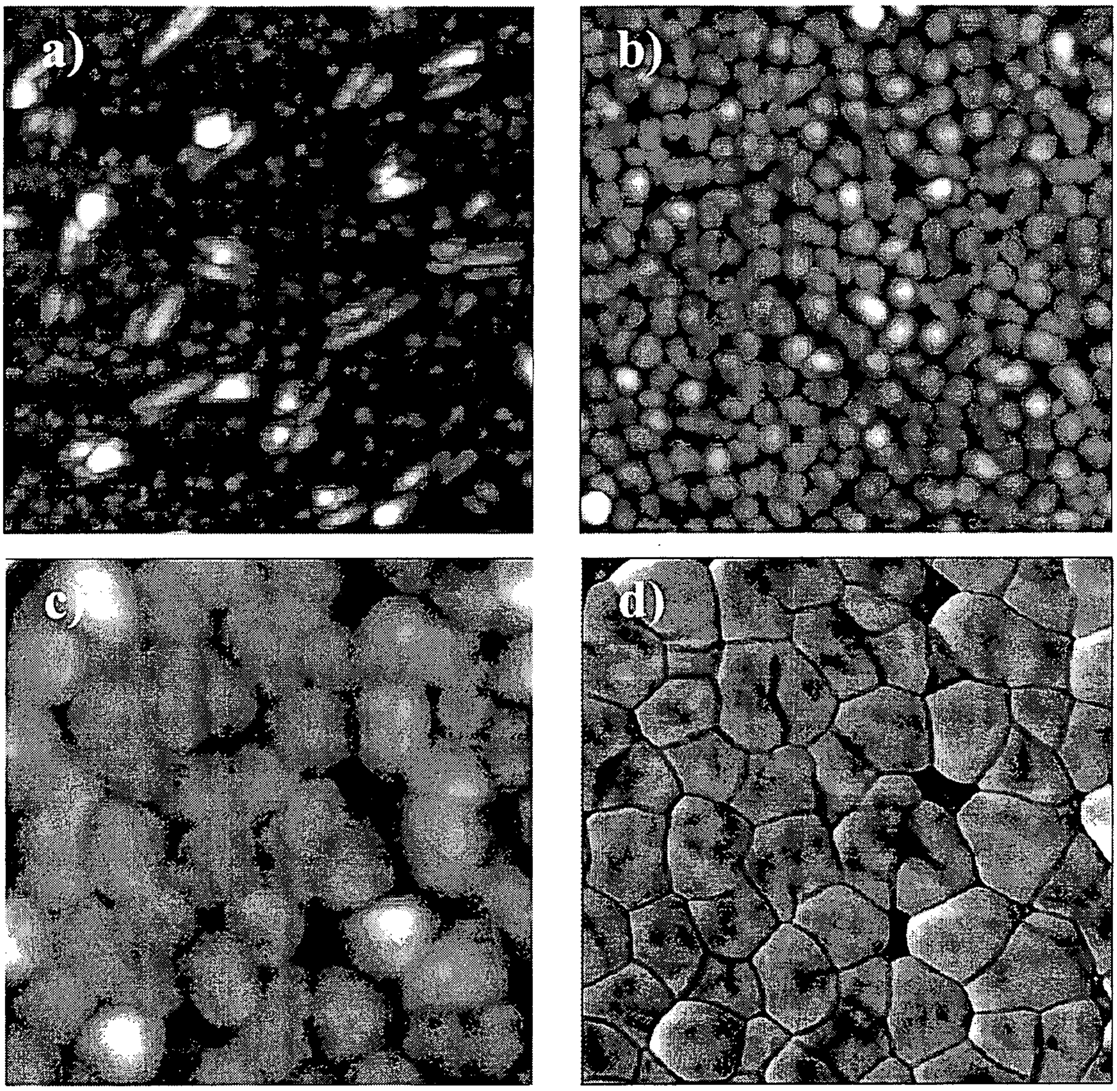


FIGURE 2

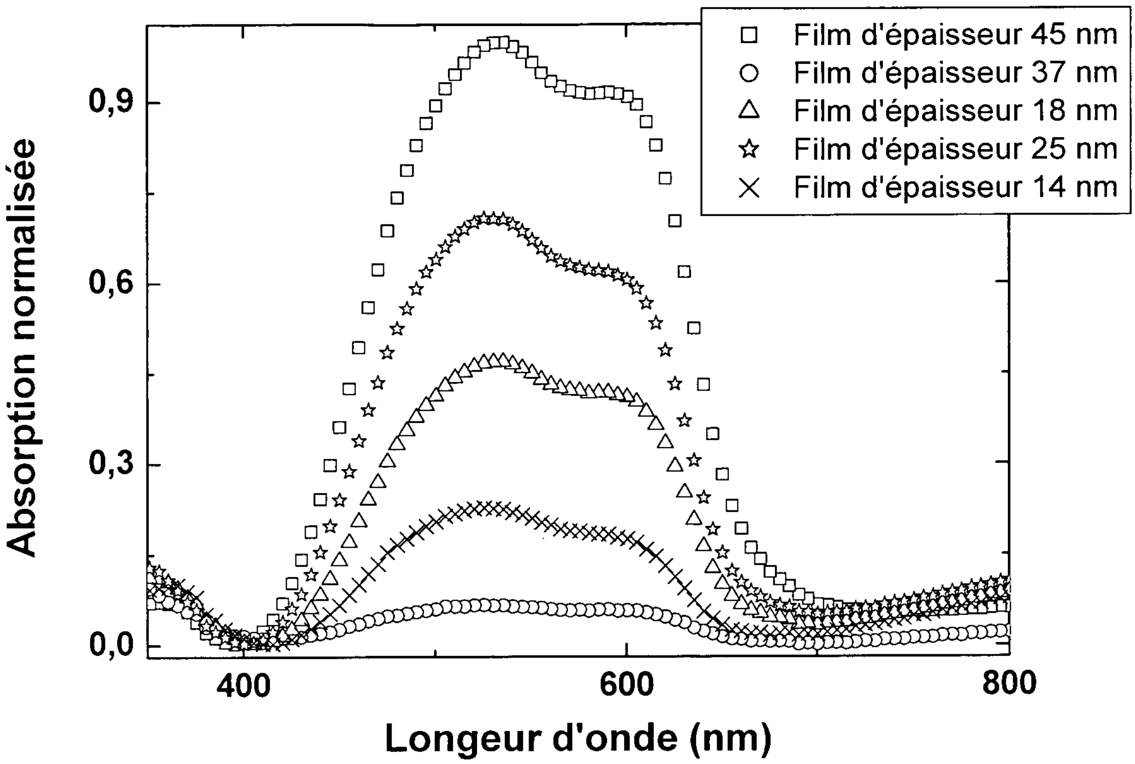
Absorption normalisée
0,0
0,3
0,6
0,9
400
600
800
Longeur d'onde (nm)
Film d'épaisseur 45 nm
Film d'épaisseur 37 nm
Film d'épaisseur 18 nm
Film d'épaisseur 25 nm
Film d'épaisseur 14 nm

FIGURE 3

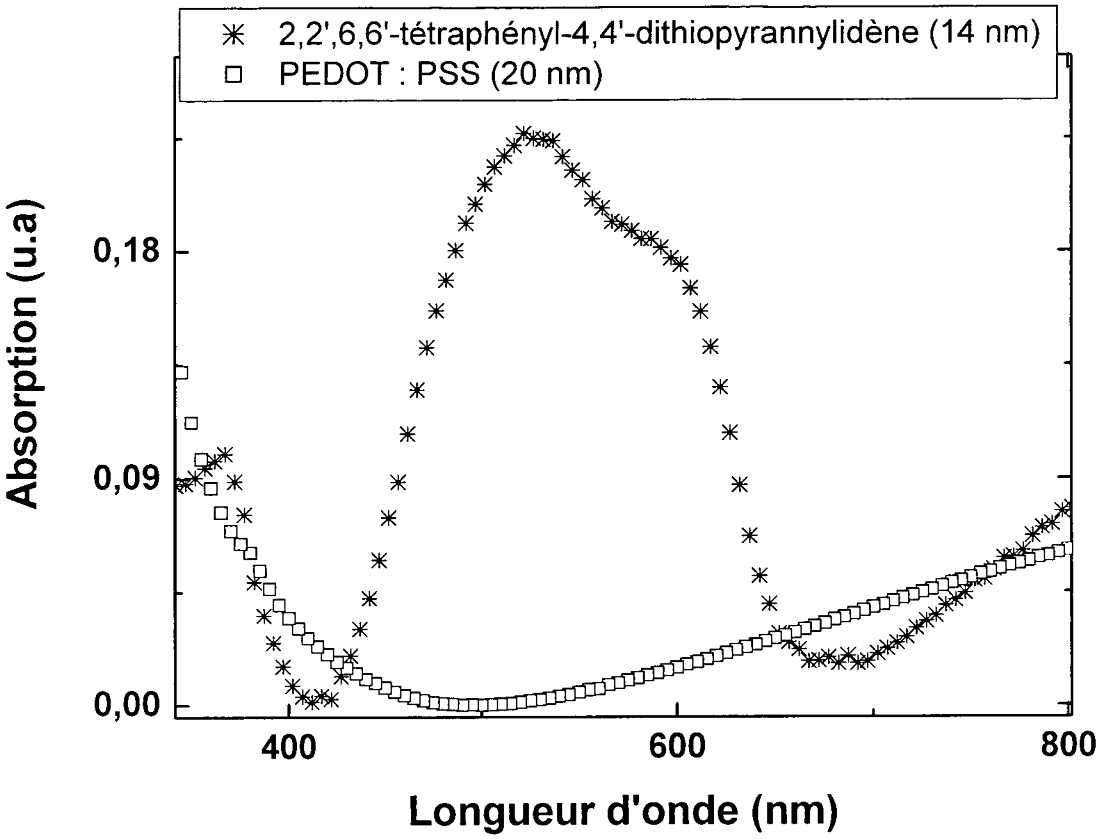
2,2',6,6'-tétraphényl-4,4'-dithiopyrannylidène (14 nm)
PEDOT : PSS (20 nm)
Absorption (u.a)
0,18
0,09
0,00
400
600
800
Longueur d'onde (nm)

FIGURE 4

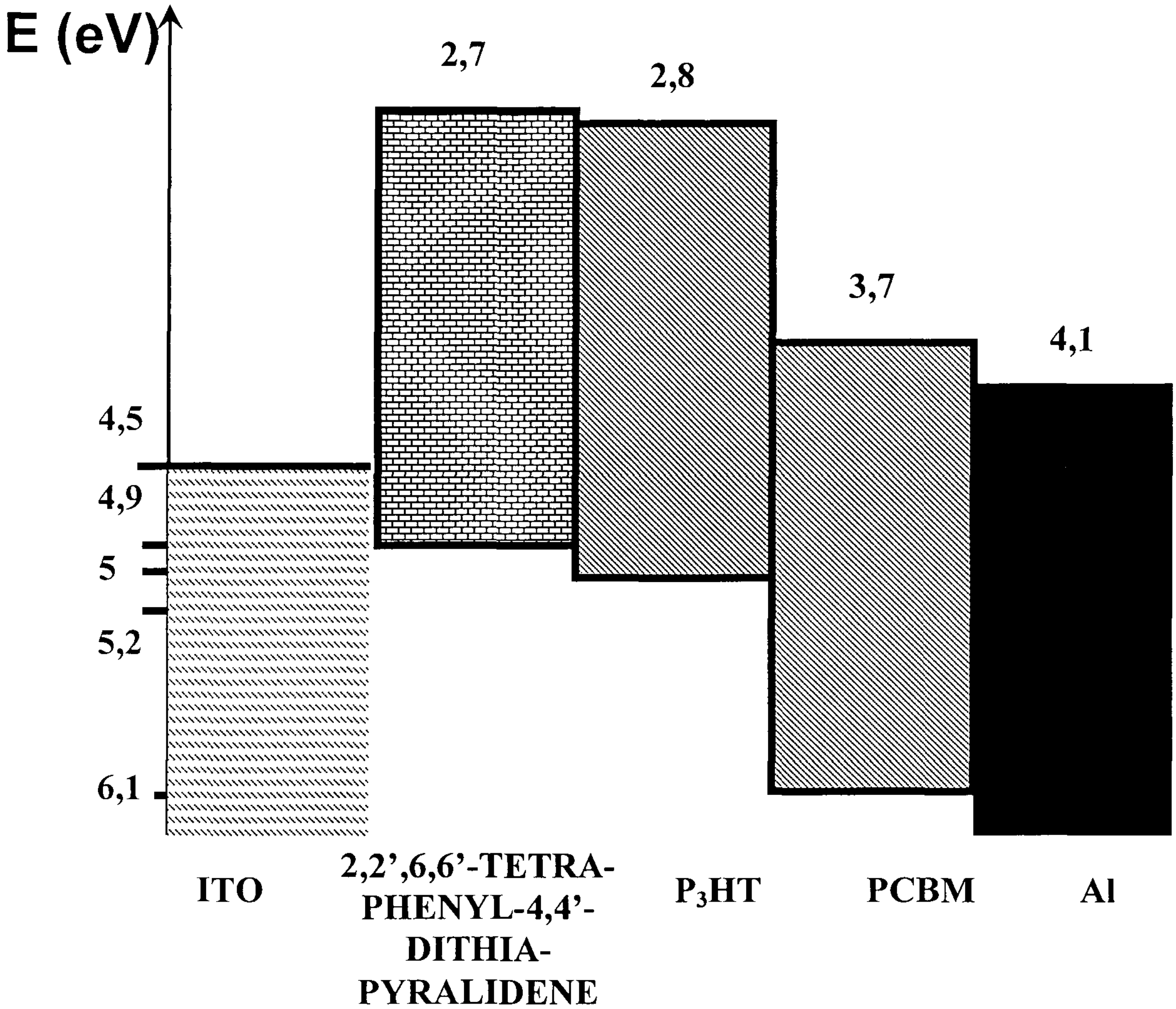
E (eV)
2,7
2,8
3,7
4,1
4,5
4,9
5
5,2
6,1
ITO
2,2',6,6'-TETRA-PHENYL-4,4'-DITHIA-PYRALIDENE
P3HT
PCBM
Al

FIGURE 5

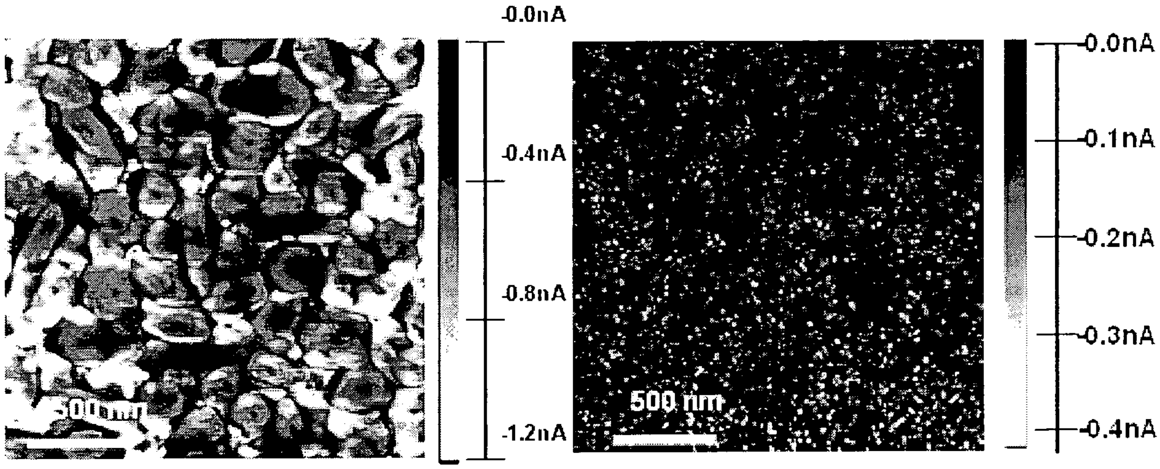


-600 -600

FIGURE 6

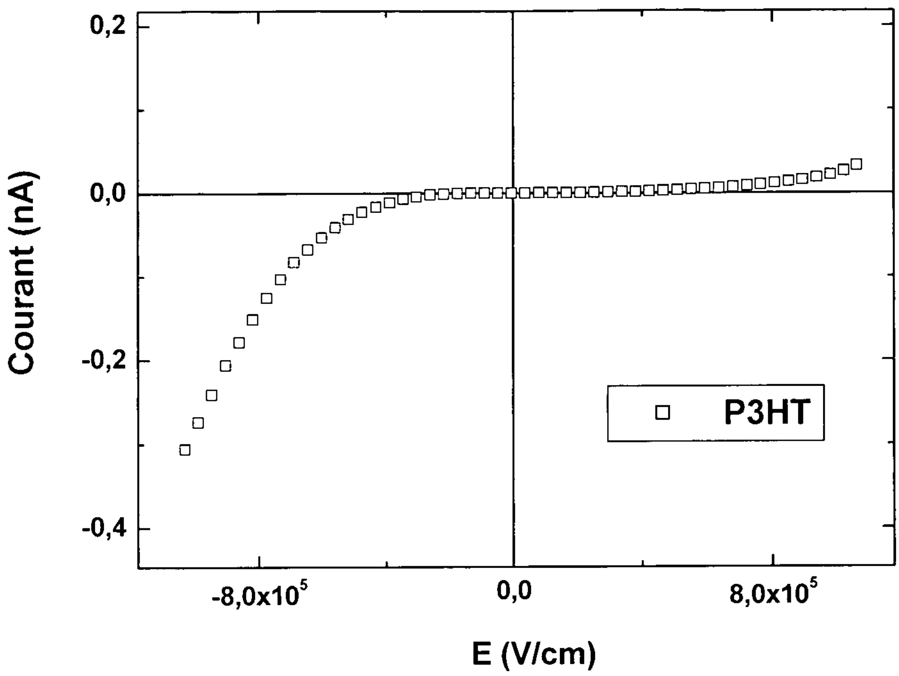


FIGURE 7

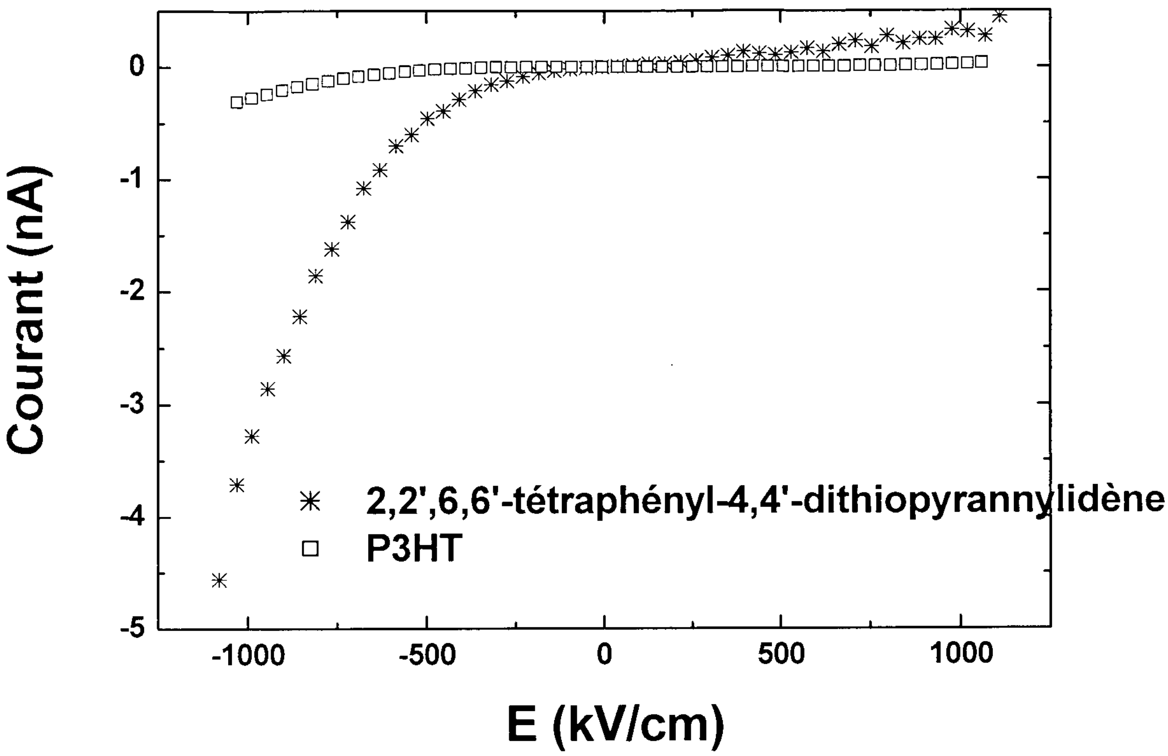
Courant (nA)
0
-1
-2
-3
-4
-5
-1000
-500
0
500
1000
E (kV/cm)
2,2',6,6'-tétraphényl-4,4'-dithiopyrannylidène
P3HT

FIGURE 8

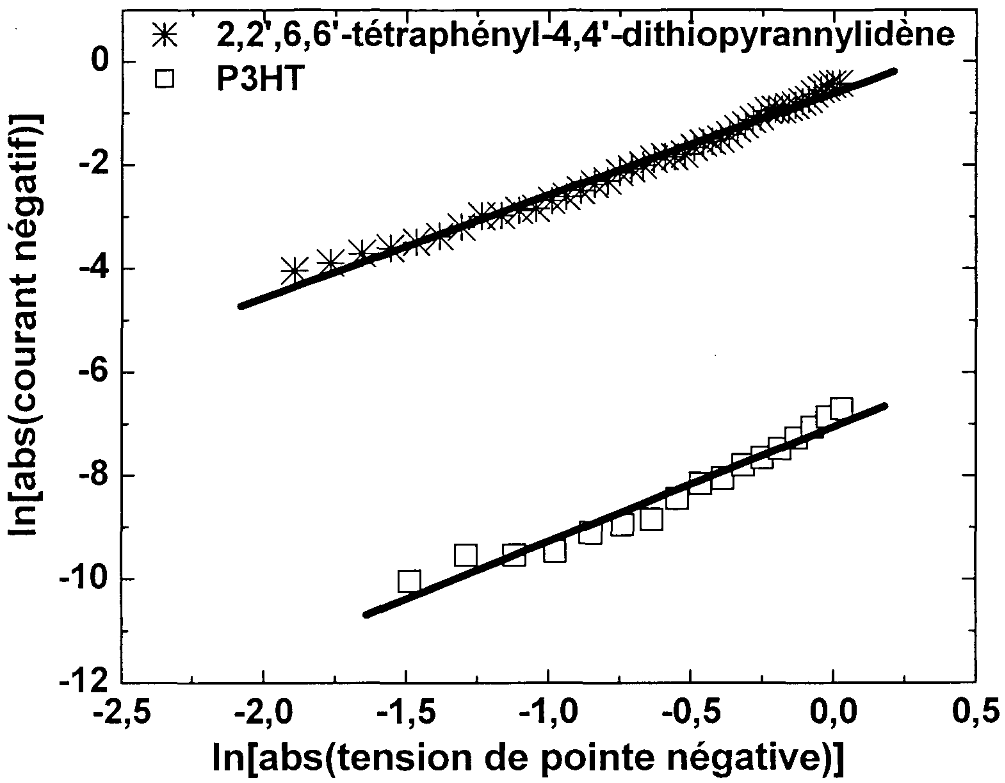
2,2',6,6'-tétraphényl-4,4'-dithiopyrannylidène
P3HT
0
-2
-4
-6
-8
-10
-12
-2,5
-2,0
-1,5
-1,0
-0,5
0,0
0,5
ln[abs(courant négatif)]
ln[abs(tension de pointe négative)]

FIGURE 9

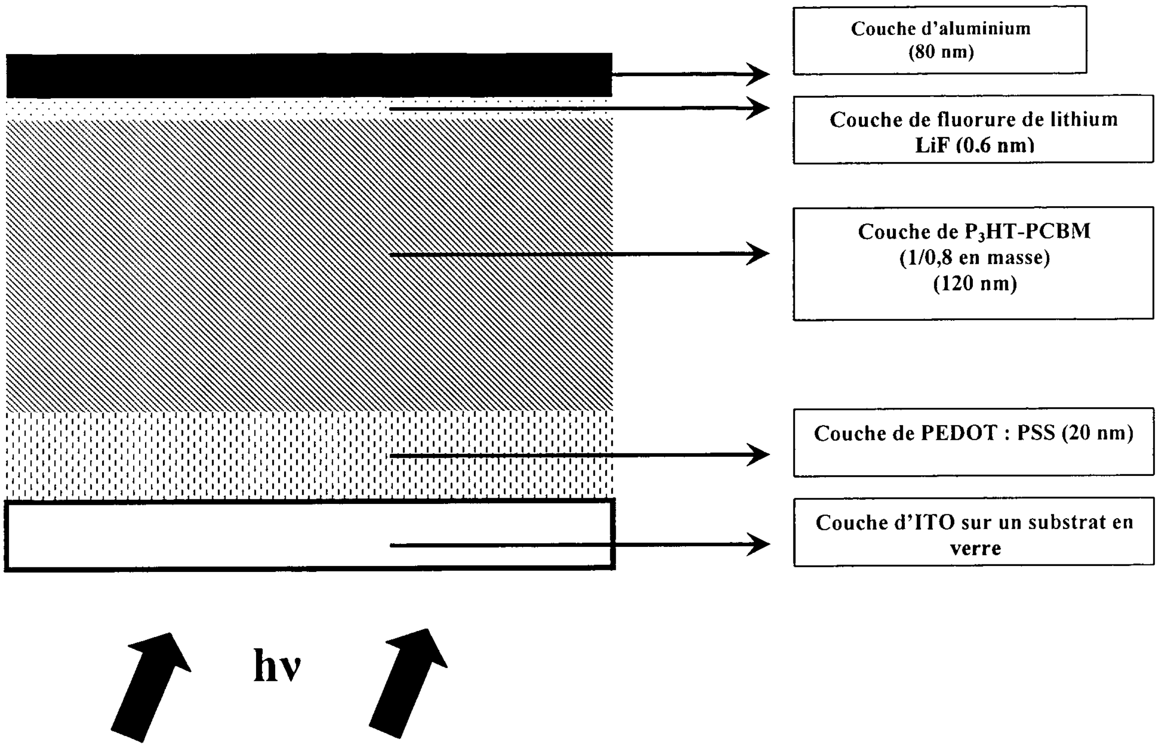
Couche d'aluminium
(80 nm)
Couche de fluorure de lithium
LiF (0.6 nm)
Couche de $P_3$HT-PCBM
(1/0,8 en masse)
(120 nm)
Couche de PEDOT : PSS (20 nm)
Couche d'ITO sur un substrat en verre
hν

FIGURE 10

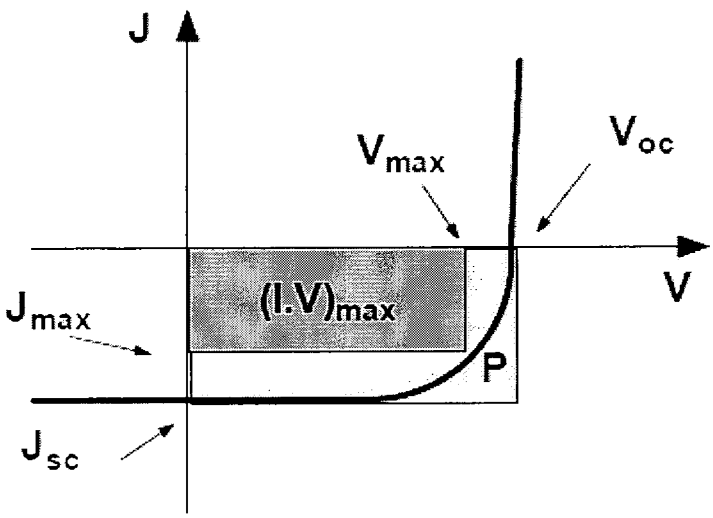


FIGURE 11

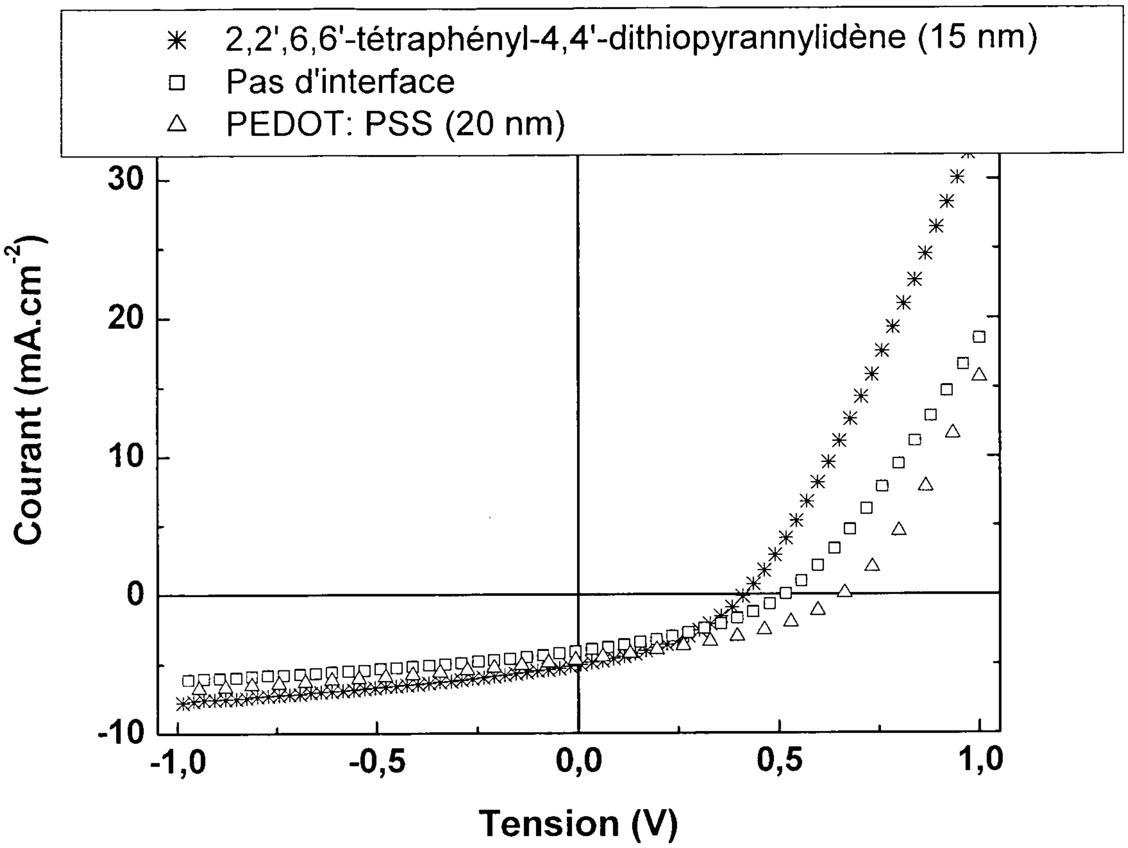
2,2',6,6'-tétraphényl-4,4'-dithiopyrannylidène (15 nm)
Pas d'interface
PEDOT: PSS (20 nm)
Courant (mA.cm-2)
30
20
10
0
-10
-1,0
-0,5
0,0
0,5
1,0
Tension (V)

FIGURE 12

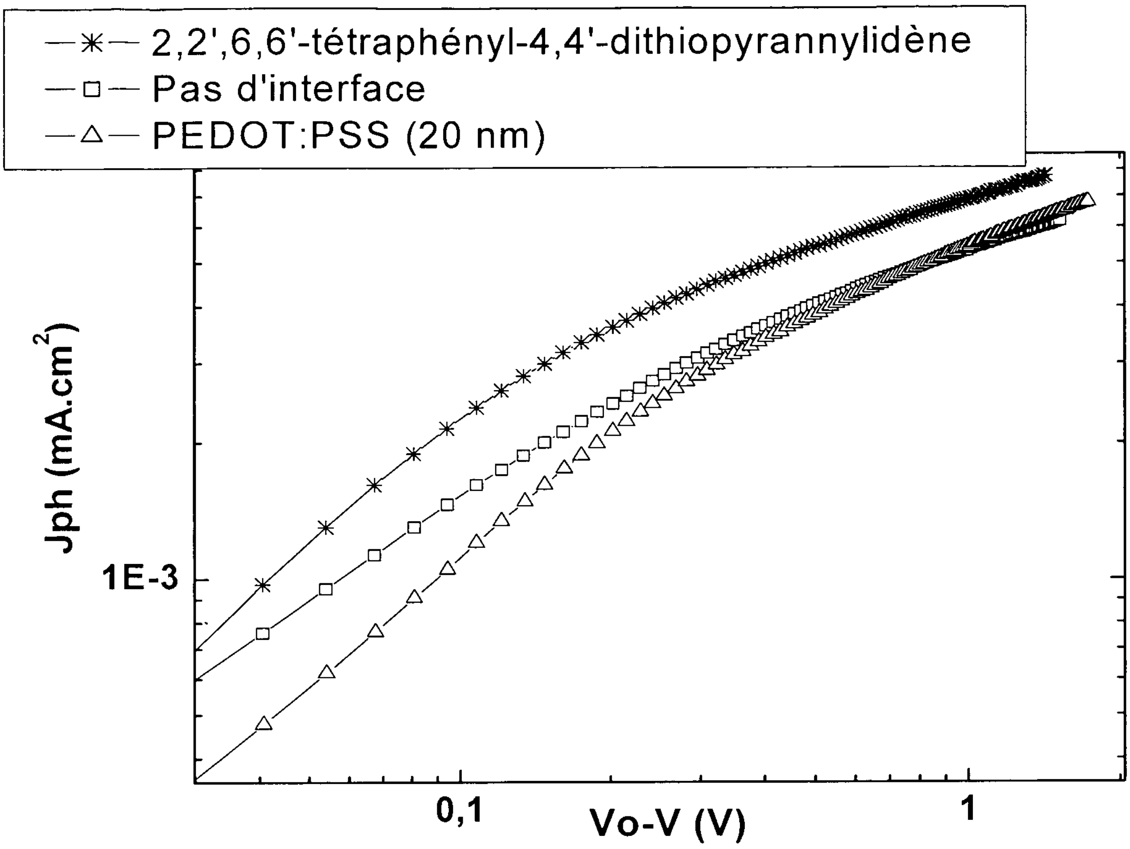
2,2',6,6'-tétraphényl-4,4'-dithiopyrannylidène
Pas d'interface
PEDOT:PSS (20 nm)
Jph (mA.cm$^2$)
1E-3
0,1
Vo-V (V)
1

FIGURE 13

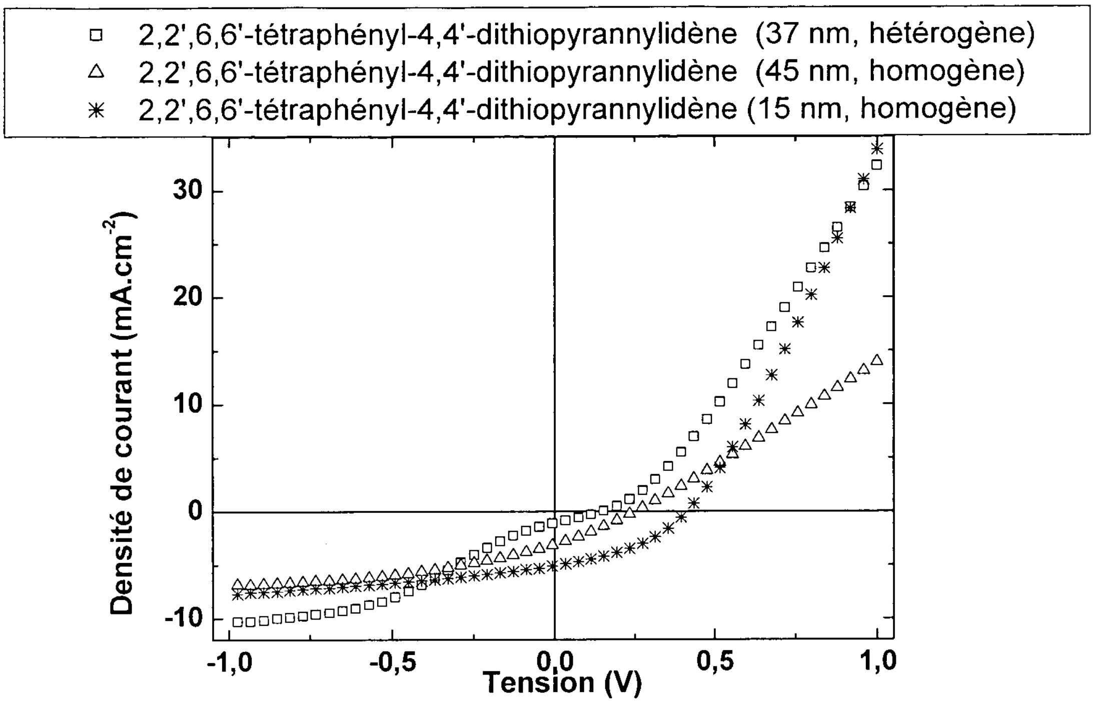
2,2',6,6'-tétraphényl-4,4'-dithiopyrannylidène (37 nm, hétérogène)
2,2',6,6'-tétraphényl-4,4'-dithiopyrannylidène (45 nm, homogène)
2,2',6,6'-tétraphényl-4,4'-dithiopyrannylidène (15 nm, homogène)
Densité de courant (mA.cm-2)
30
20
10
0
-10
-1,0
-0,5
0,0
0,5
1,0
Tension (V)

FIGURE 14

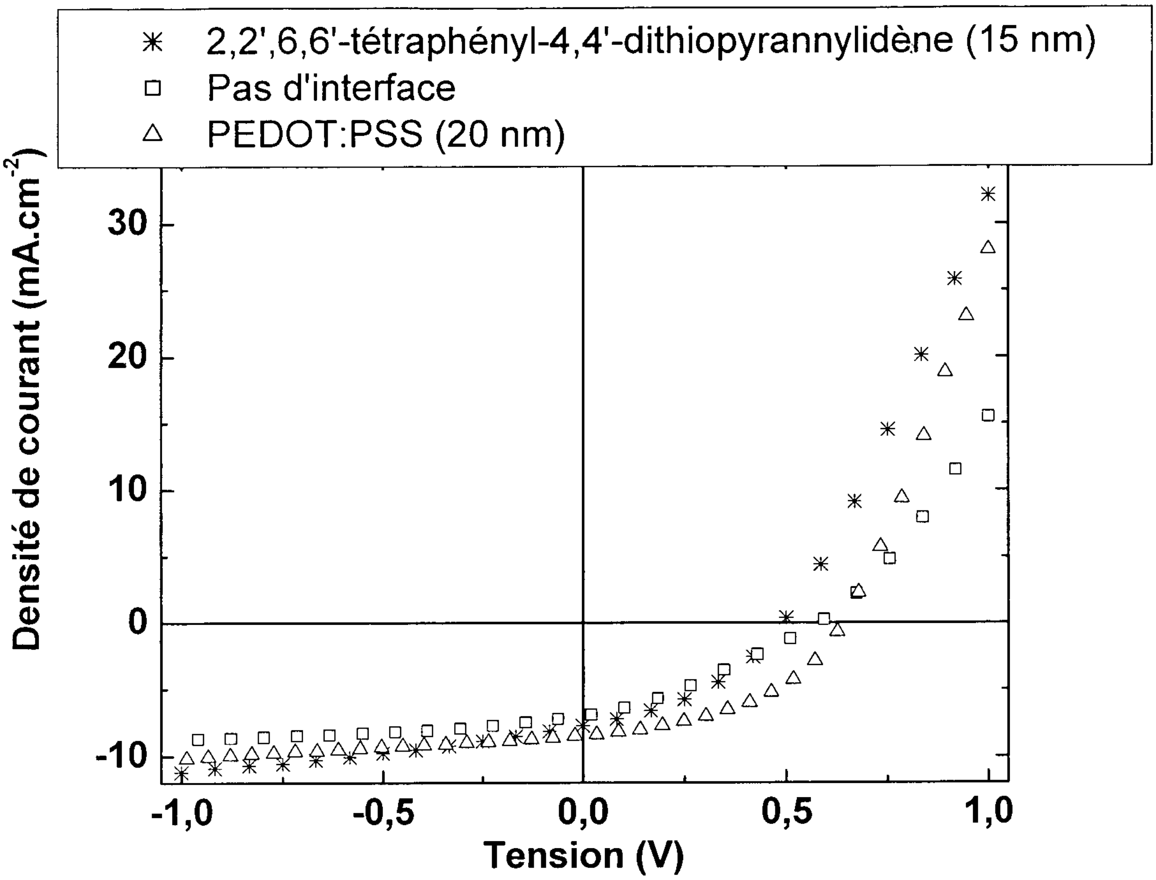
2,2',6,6'-tétraphényl-4,4'-dithiopyrannylidène (15 nm)
Pas d'interface
PEDOT:PSS (20 nm)
Densité de courant (mA.cm-2)
30
20
10
0
-10
-1,0
-0,5
0,0
0,5
1,0
Tension (V)

FIGURE 15

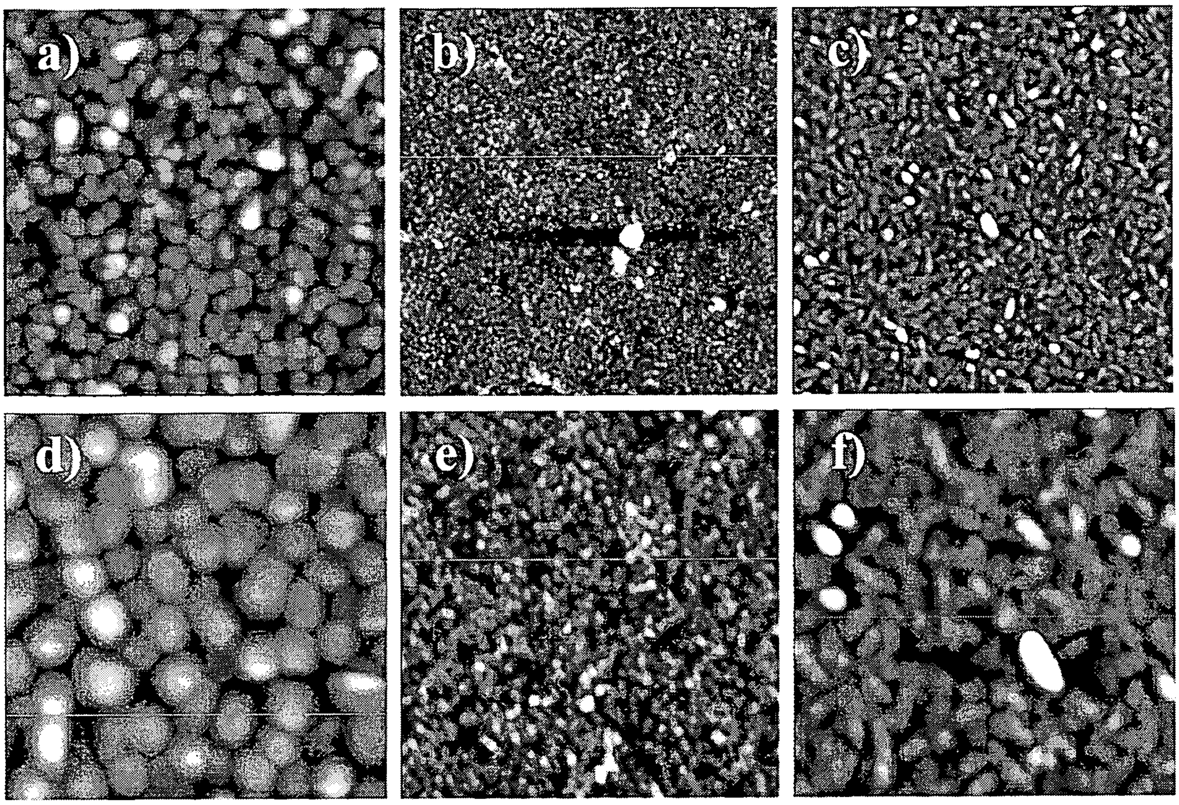


FIGURE 16

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 60264280 A


### Littérature non-brevet citée dans la description


- **CRISPIN et al.** *Journal of Polymer Science: Part B: Polymer Physics,* 2003, vol. 41, 2561-2583
- **GROENENDAAL et al.** *Adv. Mater.,* 2000, vol. 12 (7
- **CAMPBELL et al.** *Appl. Phys. Lett.,* vol. 71 (24), 3528-3530
- **KIM et al.** *Appl. Phys. Lett.,* 2008, vol. 92, 133307
- **AKKERMAN et al.** *Small,* 2008, vol. 4 (1), 100-104
- **ARMSTRONG et al.** *Thin Solid Films,* 2003, vol. 445, 342-352
- **HANSON et al.** *J. Am. Chem. Soc.,* 2005, vol. 127 (28), 10058-10062
- **KAHN et al.** *Appl. Phys. Lett.,* vol. 79 (24), 4040-4042
- **SANDMAN et al.** *J. Chem. Soc., Chem. Commun.,* 1977, vol. 687, 177-178
- **OTSUBO et al.** *J. Chem. Soc. Perkin Trans.,* 1993, 1815-1824
- **BERENJIAN et al.** *Can. J. Chem.,* 1981, vol. 59, 2612-2516
- **BOLAG et al.** *Chemistry of Materials,* 2009, vol. 21 (19), 4350-4352
- **D'ANDRADE et al.** *Organic Electronics,* 2005, vol. 6, 11-20